# EUROPEAN PATENT APPLICATION

(11) **EP 4 625 289 A1**
(43) Date of publication of application: **01.10.2025**
(21) Application number: 25165860.5
(22) Date of filing: 25.03.2025
(51) Int. Cl.: G06Q 10/0875, G06F 18/243, G06N 20/20

(54) **METHODS AND SYSTEMS FOR CLASSIFYING ANALYTE DATA**

(30) Priority: 25.03.2024 US 202463569559 P
(71) Applicant: Becton, Dickinson and Company, Franklin Lakes, New Jersey 07417-1880 (US)
(72) Inventor: Luthy, Joshua, Ocean View, Hawaii, 96737 (US)
(74) Representative: Bals & Vogel Patentanwälte PartGmbB

(57) **Abstract**

Computer-implemented methods of classifying analyte data are provided. Methods of interest include categorizing the analyte data based on analyte features associated therewith by generating a predicted class for the analyte data using a decision tree ensemble, and refining the categorized analyte data based on the analyte features and the predicted class using a distance-based classification model to classify the analyte data. Systems and non-transitory computer-readable storage media for carrying out the subject methods are also provided.

## Description

### CROSS-REFERENCE TO RELATED APPLICATION

Pursuant to 35 U.S.C. § 119 (e), this application claims priority to the filing dates of United States Provisional Patent Application Serial No. 63/569,559 filed March 25, 2024, the disclosure of which application is incorporated herein by reference in their entirety

### INTRODUCTION

The characterization of analytes in biological fluids has become an important part of biological research, medical diagnoses and assessments of overall health and wellness of a patient. Detecting analytes in biological fluids, such as human blood or blood derived products, can provide results that may play a role in determining a treatment protocol of a patient having a variety of disease conditions.

Flow cytometry is a technique used to characterize and often times sort biological material, such as cells of a blood sample or particles of interest in another type of biological or chemical sample. A flow cytometer typically includes a sample reservoir for receiving a fluid sample, such as a blood sample, and a sheath reservoir containing a sheath fluid. The flow cytometer transports the particles (including cells) in the fluid sample as a cell stream to a flow cell, while also directing the sheath fluid to the flow cell. To characterize the components of the flow stream, the flow stream is irradiated with light. Variations in the materials in the flow stream, such as morphologies or the presence of fluorescent labels, may cause variations in the observed light and these variations allow for characterization and separation. To characterize the components in the flow stream, light must impinge on the flow stream and be collected. Light sources in flow cytometers can vary and may include one or more broad spectrum lamps, light emitting diodes as well as single wavelength lasers. The light source is aligned with the flow stream and an optical response from the illuminated particles is collected and quantified.

Isolation of biological particles has been achieved by adding a sorting or collection capability to flow cytometers. Particles in a segregated stream, detected as having one or more desired characteristics, are individually isolated from the sample stream by mechanical or electrical removal. A common flow sorting technique utilizes drop sorting in which a fluid stream containing linearly segregated particles is broken into drops. The drops containing particles of interest are electrically charged and deflected into a collection tube by passage through an electric field. Typically, the linearly segregated particles in the stream are characterized as they pass through an observation point situated just below the nozzle tip. Once a particle is identified as meeting one or more desired criteria, the time at which it will reach the drop break-off point and break from the stream in a drop can be predicted. Ideally, a brief charge is applied to the fluid stream just before the drop containing the selected particle breaks from the stream and then grounded immediately after the drop breaks off. The drop to be sorted maintains an electrical charge as it breaks off from the fluid stream, and all other drops are left un-charged.

The parameters measured using a flow cytometer typically include light at the excitation wavelength scattered by the particle in a narrow angle along a mostly forward direction, referred to as forward-scatter (FSC), the excitation light that is scattered by the particle in an orthogonal direction to the excitation laser, referred to as side-scatter (SSC), and the light emitted from fluorescent molecules in one or more detectors that measure signal over a range of spectral wavelengths, or by the fluorescent dye that is primarily detected in that specific detector or array of detectors. Different cell types can be identified by their light scatter characteristics and fluorescence emissions resulting from labeling various cell proteins or other constituents with fluorescent dye-labeled antibodies or other fluorescent probes.

Flow cytometers may further comprise means for recording the measured data and analyzing the data. For example, data storage and analysis may be carried out using a computer connected to the detection electronics. For example, the data can be stored in tabular form, where each row corresponds to data for one particle, and the columns correspond to each of the measured features. The use of standard file formats, such as an "FCS" file format, for storing data from a particle analyzer facilitates analyzing data using separate programs and/or machines. Using current analysis methods, the data typically are displayed in 1-dimensional histograms or 2-dimensional (2D) plots for ease of visualization, but other methods may be used to visualize multidimensional data.

While flow cytometer data generally contains numerous data points (i.e., events), it is often the case that only a certain portion of the flow cytometer data is of interest to the user. For example, it may be desirable to identify the best parameters to discriminate debris/small particles from single cells and multiplets. Debris are essentially pieces of cells that have been broken during processing. Multiplets are two or more cells that are joined together. Cellular debris can be considered as 'junk' or data that users do not want to collect or process with further analyses. Multiplets are also events that are desirable to remove from analysis as the fluorescent signal obtained from these are double of what would be observed from single cells, i.e., they are outlier events. Removal of such debris and multiplets is often a first step performed in the analysis of flow data.

### SUMMARY

The present disclosure provides improvements to the processes by which analyte data (e.g., flow cytometer data) is classified, e.g., in the process of removing data associated with undesirable analytes (e.g., debris, multiplets, etc.). In particular, it was realized that analyte classification often varies greatly between different users, thereby hindering generalizability and reproducibility of results. As such, a simplified process for data cleanup is desirable. Particularly, automated processes are needed for cleaning data that minimize the removal of events of interest which can result from the drawing of manual gates. Embodiments of the present disclosure satisfy these and other needs.

Aspects of the disclosure include computer-implemented methods of classifying analyte data. Methods of interest include categorizing the analyte data based on analyte features associated therewith by generating a predicted class for the analyte data using a decision tree ensemble (e.g., random forest classification model), and refining the categorized analyte data based on the analyte features and the predicted class using a distance-based classification model (e.g., k-nearest neighbors classifier) to classify the analyte data. In certain cases, the distance of the distance-based classifier is selected from a Manhattan distance, a Euclidean distance, a Chebyshev distance and a cosine distance. In embodiments, the method comprises refining the predicted classes of the categorized analyte data using a vantage-point tree, a k-dimensional tree, ball tree, cover tree, locality-sensitive hashing, hierarchical navigable small world, approximate nearest neighbors with random projection trees, GPU-based KNN search, or a brute force KNN search. While analyte data may vary, in some cases the analyte data is flow cytometer data. In some such cases, the method comprises generating the flow cytometer data using a flow cytometer. Predicted classes and/or classifications that may be assigned to the data can include, e.g., debris, single cells and aggregates. In some cases, analyte features include size features, imaging features, and scatter features (e.g., side-scatter (SSC) features and forward-scatter (FSC) features). In certain instances, analyte features include fluorescent features. In some such instances, methods include classifying the analyte data into subgroups based on the fluorescent features. In embodiments, the method includes classifying the analyte data based on from 4 to 30 analyte features. In some implementations, methods include ranking the analyte features by importance (e.g., by calculating an ANOVA F-value). Additionally, methods may in some versions include training the decision tree ensemble using analyte features from a training dataset. This may in certain instances include also training the distance-based classification model using the analyte features from the training dataset and the predicted class. Methods according to some embodiments further include producing an image of the classified analyte data, such as by rendering a gate around the classified analyte data.

Aspects of the disclosure also include systems. Systems of interest include a memory operably coupled to a processor, wherein the memory comprises instructions stored thereon, which when executed by the processor, cause the processor to carry out the methods of the disclosure, e.g., as described above and herein. In some embodiments, the processor of the subject systems is operably connected to one or more flow cytometers. Aspects of the disclosure also include non-transitory computer-readable storage media comprising instructions stored thereon for classifying analyte data by a method of the disclosure, e.g., as described above and herein.

### BRIEF DESCRIPTION OF THE FIGURES

The disclosure may be best understood from the following detailed description when read in conjunction with the accompanying drawings. Included in the drawings are the following figures:
**FIG. 1A-1B** depicts a flow diagram for practicing methods according to certain embodiments.
**FIG. 2** depicts a system according to certain embodiments.
**FIG. 3** presents a block diagram of a computer-controlled system according to certain embodiments.
**FIG. 4** presents a scatter plot of two flow cytometer data features.
**FIG. 5** presents a scatter plot of two scaled flow cytometer data features.
**FIG. 6** presents a bar graph showing feature importance in a random forest model.
**FIG. 7** presents a confusion matrix for assessing a classification model.
**FIG. 8** presents a confusion matrix for assessing a classification model.
**FIG. 9** presents a confusion matrix for assessing a classification model.

### DETAILED DESCRIPTION

Computer-implemented methods of classifying analyte data are provided. Methods of interest include categorizing the analyte data based on analyte features associated therewith by generating a predicted class for the analyte data using a decision tree ensemble, and refining the categorized analyte data based on the analyte features and the predicted class using a distance-based classification model to classify the analyte data. Systems and non-transitory computer-readable storage media for carrying out the subject methods are also provided.

Before the present invention is described in greater detail, it is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure. Any recited method can be carried out in the order of events recited or in any other order which is logically possible.

While the system and method has or will be described for the sake of grammatical fluidity with functional explanations, it is to be expressly understood that the claims, unless expressly formulated under 35 U.S.C. §112, are not to be construed as necessarily limited in any way by the construction of "means" or "steps" limitations, but are to be accorded the full scope of the meaning and equivalents of the definition provided by the claims under the judicial doctrine of equivalents, and in the case where the claims are expressly formulated under 35 U.S.C. §112 are to be accorded full statutory equivalents under 35 U.S.C. §112.

### METHODS OF CLASSIFYING ANALYTE DATA

Aspects of the disclosure include computer-implemented methods of classifying analyte data. By "analyte data", it is meant data obtained by assessing a particular analyte for certain characteristics. By "classifying" the analyte data, it is meant designating analyte data (e.g., groups of analyte data) as belonging to a particular type out of one or more possible different types said data could belong to. Methods of the disclosure may in some cases be sufficient to improve analyte data classification relative to conventional classification methods, such as where analyte data is manually classified by a user (e.g., by drawing a gate on flow cytometer data). For example, the subject methods may in certain embodiments increase classification accuracy. Accuracy may be determined by assessing whether each analyte or data point/event associated therewith does in fact belong to the particular type with which it is classified. In certain cases, methods of the disclosure may increase classification accuracy relative to conventional methods (e.g., drawing a manual gate) by 1% or more, such as 5 % or more, such as 10% or more, such as 15% or more and including 20% or more. In embodiments, practicing the subject methods is sufficient to increase the speed and/or efficiency with which analyte data is classified relative to conventional methods (e.g., drawing a manual gate) such as by 1% or more such as 5% or more, such as 10% or more, such as 15% or more and including 20% or more.

Methods of the disclosure include categorizing the analyte data based on analyte features associated therewith. In some cases, the analyte data is flow cytometer data. By "flow cytometer data" it is meant information regarding the characteristics of sample particles that has been collected by any number of detectors in a particle analyzer. As discussed herein, a "particle analyzer" is an analytical tool (e.g., flow cytometer) that enables the characterization of particles on the basis of certain (e.g., optical) parameters. By "particle", it is meant a discrete component of a biological sample such as a molecule, analyte-bound bead, individual cell, or the like.

Flow cytometer data may be received from any suitable source. In some embodiments, flow cytometer data is received from the memory of a storage device. In such embodiments, flow cytometer data may have been previously generated and saved in the memory of the storage device for subsequent recall and analysis. In other embodiments, the flow cytometer data is received in real time. Put another way, flow cytometer data generated during the operation of a flow cytometer may subsequently (e.g., immediately) populate the data-space (e.g., two-dimensional plot). In embodiments, the flow cytometer data is received from a forward scatter detector. A forward scatter detector may, in some instances, yield information regarding the overall size of a particle. In embodiments, the flow cytometer data is received from a side scatter detector. A side scatter detector may, in some instances, be configured to detect refracted and reflected light from the surfaces and internal structures of the particle, which tends to increase with increasing particle complexity of structure.

In certain embodiments, the particles are detected and uniquely identified by exposing the particles to excitation light and measuring the fluorescence of each particle in one or more detection channels, as desired. Fluorescence emitted in detection channels used to identify the particles and binding complexes associated therewith may be measured following excitation with a single light source, or may be measured separately following excitation with distinct light sources. If separate excitation light sources are used to excite the particle labels, the labels may be selected such that all the labels are excitable by each of the excitation light sources used. In embodiments, the flow cytometer data is received from a fluorescent light detector. A fluorescent light detector may, in some instances, be configured to detect fluorescence emissions from fluorescent molecules, e.g., labeled specific binding members (such as labeled antibodies that specifically bind to markers of interest) associated with the particle in the flow cell. In certain embodiments, methods include detecting fluorescence from the sample with one or more fluorescence detectors, such as 2 or more, such as 3 or more, such as 4 or more, such as 5 or more, such as 6 or more, such as 7 or more, such as 8 or more, such as 9 or more, such as 10 or more, such as 15 or more and including 25 or more fluorescence detectors. In embodiments, each of the fluorescence detectors is configured to generate a fluorescence data signal. Fluorescence from the sample may be detected by each fluorescence detector, independently, over one or more of the wavelength ranges of 200 nm - 1200 nm. In some instances, methods include detecting fluorescence from the sample over a range of wavelengths, such as from 200 nm to 1200 nm, such as from 300 nm to 1100 nm, such as from 400 nm to 1000 nm, such as from 500 nm to 900 nm and including from 600 nm to 800 nm. In other instances, methods include detecting fluorescence with each fluorescence detector at one or more specific wavelengths. For example, the fluorescence may be detected at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof, depending on the number of different fluorescence detectors in the subject light detection system. In certain embodiments, methods include detecting wavelengths of light which correspond to the fluorescence peak wavelength of certain fluorophores present in the sample. In embodiments, flow cytometer data is received from one or more light detectors (e.g., one or more detection channels), such as 2 or more, such as 3 or more, such as 4 or more, such as 5 or more, such as 6 or more and including 8 or more light detectors (e.g., 8 or more detection channels).

In some cases, prior to categorizing the analyte data, methods include preprocessing the data, e.g., such that it is in a more suitable form for manipulation by different models. Any suitable preprocessing protocol may be employed. In some embodiments, methods include standardizing analyte features, e.g., such that they are centered around the mean and scaled to unit variance.

As noted above, methods of the disclosure include categorizing the analyte data based on analyte features associated therewith. By "analyte features" it is meant one or more properties (e.g., optical, impedance, and/or temporal properties) associated with each individual analyte (e.g., particle) such that each analyte is present in the analyte data as a set of digitized feature values. Depending on the requirements of a given experiment, the number of analyte features present in the data may vary and can include, e.g., 10 features or more, such as 20 features or more, such as 30 features or more, such as 40 features or more, such as 50 features or more, and including 60 features or more. In certain instances, the analyte features are selected from size features, imaging features, and scatter features. In some such instances the analyte features are scatter features selected from side-scatter (SSC) features and forward-scatter (FSC) features. Where the analyte data is flow cytometer data, the analyte features may also be associated with and/or obtained from fluorescent light, axial light loss (ALL), and the like. Exemplary features include, but are not limited to, size, center of mass, short axis moment, diffusivity, long axis moment, radial moment, maximum intensity, and eccentricity.

The number and type of analyte features used to classify analyte data may in some cases vary. In select versions, the number and type of analyte features used to classify analyte data are tunable parameters that can be optimized throughout the use of the present disclosure (e.g., during model training). In some instances, the method comprises classifying the analyte data based on from 3 to 50 analyte features, such as from 4 to 30 analyte features, such as 4 to 25 analyte features, such as 4 to 15 analyte features, and including from 4 to 10 analyte features. In certain embodiments, the method comprises classifying the analyte data based on 3 or more analyte features, such as 4 or more analyte features, such as 10 or more analyte features, such as 20 or more analyte features, such as 25 or more analyte features, and including 30 or more analyte features. In some implementations, use of a number of analyte features in the above-described ranges will generate suitably accurate and precise classifications. Furthermore, in some cases, methods include selecting only a subset of available analyte features for use in analyte classification. In some such cases, methods include ranking the analyte features by importance. In other words, methods may involve determining which analyte features are more strongly correlated with particular classifications such that possession of a particular analyte feature or combination thereof is suitably associated with a given classification. Any suitable method ranking features in this manner may be employed. In select versions, ranking the analyte features by importance comprises calculating an analysis of variance (ANOVA) F-value. This value measures the difference in means between groups relative to the variation within the groups, and is suitable for both positive and negative values.

In some embodiments, methods include generating one or more population clusters based on the analyte features (e.g., particles, nucleic acids, etc.) in the sample. As used herein, a "population", or "subpopulation" of analytes, such as cells, nucleic acids or other particles, generally refers to a group of analytes that possess properties (e.g., optical, impedance, or temporal properties) with respect to one or more measured parameters such that measured parameter data form a cluster in the data space. In embodiments, data is comprised of signals from any given number of different parameters, such as, for instance 2 or more, 3 or more, 4 or more, 5 or more, 6 or more, 7 or more, 8 or more, 9 or more, 10 or more, and including 20 or more. Thus, populations are recognized as clusters in the data. Conversely, each data cluster generally is interpreted as corresponding to a population of a particular type of cell or analyte, although clusters that correspond to noise or background typically also are observed. A cluster may be defined in a subset of the dimensions, e.g., with respect to a subset of the measured parameters, which corresponds to populations that differ in only a subset of the measured parameters or features extracted from the measurements of the cell, particle or nucleic acid.

In embodiments, methods include receiving data, calculating parameters of each analyte, and clustering together analytes based on the calculated parameters. For example, where the data is flow cytometer data, an experiment may include particles labeled by several fluorophores or fluorescently labeled antibodies, and groups of particles may be defined by populations corresponding to one or more fluorescent measurements. In the example, a first group may be defined by a certain range of light scattering for a first fluorophore, and a second group may be defined by a certain range of light scattering for a second fluorophore. If the first and second fluorophores are represented on an x and y axis, respectively, two different color-coded populations might appear to define each group of particles, if the information was to be graphically displayed. Any number of analytes may be assigned to a cluster, including 5 or more analytes, such as 10 or more analytes, such as 50 or more analytes, such as 100 or more analytes, such as 500 analytes and including 1000 analytes. In certain embodiments, the method groups together in a cluster rare events (e.g., rare cells in a sample, such as cancer cells) detected in the sample. In these embodiments, the analyte clusters generated may include 10 or fewer assigned analytes, such as 9 or fewer and including 5 or fewer assigned analytes.

Methods of the disclosure further include categorizing the analyte data by generating a predicted class for the analyte data using a decision tree ensemble. By "predicted class" it is meant a projected classification of the analyte data. The predicted class is considered to be provisional and is subject to revision in a refining step (described in greater detail below). The predicted class may any category that is currently understood by one of ordinary skill in the art to be associated with the given analyte data (e.g., flow cytometer data), or has yet to be developed. In some cases, involving flow cytometry, the predicted class is related to the identity of a substance associated with a given event (i.e., an entity detected and analyzed at a given time by the flow cytometer) as a particle. In other words, the predicted class may indicate whether the event corresponds to an individual particle, an aggregate of particles (e.g., doublet, triplet), or something else entirely. For example, in some instances, the predicted class is an individual particle, such as a single cell. In other cases, the predicted class is an aggregate. In some such cases, the aggregate may include 2 or more particles, 3 or more particles, 4 or more particles, and including 5 or more particles. In other words, the aggregate may be considered a doublet, a triplet, a quadruplet, and so on, as appropriate depending on the number of particles comprising the aggregate. Additionally, the predicted class may be debris. "Debris" may represent any substance that is not of interest for analysis and can include, for example, components of lysed and/or dead cells (e.g., organelles, etc.). In certain cases, the predicted class is selected from debris, single cells and aggregates. Methods of the disclosure may include categorizing the analyte data into multiple different predicted classes. For example, a first population of analyte data may be categorized as single cells, a second population of flow cytometer data may be categorized as aggregates, and a third population of flow cytometer data may be categorized as debris.

In alternative or additional cases, the predicted class is associated with a phenotype of the analyte(s) (e.g., particles). Phenotypes may be determined based on the positivity or negativity of the flow cytometer data in the relevant population or subpopulation with respect to any number of different parameters. For example, where the analyzed particles include one or more fluorochromes, the phenotype of a population of flow cytometer data may be determined by assessing the positivity or negativity of the group of particles with respect to each fluorochrome. In such cases, it can be said that the analyte features comprise fluorescent features. Methods according to such embodiments may include classifying the analyte data into subgroups based on the fluorescent features. In certain embodiments, populations of flow cytometer data are assigned a predicted class based on their status relative to a hierarchy. A "hierarchy" as described herein defines the criteria by which flow cytometer data is grouped into a particular population and associated with a phenotype. In some embodiments, the hierarchy establishes the shared characteristics of data points that are positive or negative for the same parameters. For example, a hierarchy for clustering T cells might proceed by determining the positivity or negativity of the cells with respect to the presence of CD4 and CD8. A cell that is positive for CD4 but negative for CD8 is a "CD4 T Cell", while a cell that is positive for both markers is a "Double Positive T Cell", and so forth.

As noted above, the predicted class is generated using a decision tree ensemble. As discussed herein, a "decision tree ensemble" refers to a machine learning technique whereby multiple decision trees are employed to make a classification. As is understood in the art of machine learning, a "decision tree" refers to a mechanism for determining a classification for an entity given a set of observations of that entity, the mechanism employing leaves representing a predicted class and branches that represent conjunctions of features leading to those predicted classes. Ensemble techniques that may be adapted for use in the present methods include, but are not limited to, boosted tree ensembles, bootstrap aggregated (i.e., bagged) ensembles, and rotation forest ensembles. In some instances, the decision tree ensemble is comprised of a random forest classification model. As is understood in the art, a "random forest classification model" employs a plurality of decision trees at training time. The output of the random forest is the predicted class selected by the most trees given the observations provided as input (i.e., analyte features). The present inventor has realized that random forest is very effective for datasets with complex, non-linear relationships. In addition, use of the random forest provides insights into feature importance, which is valuable for understanding the model. Due to the ensemble nature (bagging), random forests are also less prone to overfitting than individual decision trees. Moreover, random forests work well for both classification and regression tasks. In some cases, the random forest classification model is an enriched random forest (ERF) employing weighted random sampling of the training data. In alternative cases, the random forest classification model is a tree-weighted random forest (TWRF) in which the trees are weighted differently. In other cases, decision tree ensemble is a gradient boosting classification model. As is understood in the art, a "gradient boosting classification model" employs independent decision trees that are built sequentially based on the errors of the previous trees.

Methods of the disclosure additionally include refining the categorized analyte data based on the analyte features and the predicted class using a distance-based classification model. By "refining" the categorized analyte data it is meant receiving the predicted classes from the decision tree ensemble, and carrying out adjustments to these classes, e.g., to ensure their precision and accuracy. Put another way, the predicted classes, e.g., in the form of a data column, is received by the distance-based classification model along with the features such that the predicted classes essentially constitute an additional feature. In some instances, refining the predicted classes involves maintaining some classes while changing others. The "distance" in the distance-based classification model may vary, and may in some cases be adjusted using parameter tuning. In some cases, the distance is a Euclidean distance, i.e., length of a line segment between the two points. In other cases, the distance is a Manhattan distance in which the distance between two points is the sum of the absolute differences of their Cartesian coordinates. In still other cases, the distance is a Chebyshev distance, i.e., the greatest of the differences between two vectors along any coordinate dimension. In yet other cases, the distance is a Minkowski distance, i.e., a generalization of the Euclidian distance and the Manhattan distance. In still other cases, the distance is a cosine distance, i.e., the complement of cosine similarity. In certain instances, the distance is selected from Euclidean distance, Manhattan distance, Chebyshev distance, Minkowski distance and cosine distance. Distance-based classification models that may be employed may also vary. In some cases, the distance-based classification model is comprised of a learning vector quantizaton (LVQ) classifier. LVQ involves a winner-takes-all Hebbian-learning-based approach. In additional cases, the distance-based classification model is comprised of a self-organizing-map (SOM) classifier. SOMs are algorithms for unsupervised learning configured to cause different parts of the network to respond similarly to certain input patterns. In further cases, the distance-based classification model is comprised of a *k-*means clustering model which partitions observations into k clusters, in which each observation belongs to the cluster with the nearest mean. In still further cases, the distance-based classification model is a *k*-nearest neighbors (KNN) classifier. KNN classifiers work using a plurality vote of neighbors, with a relevant event being assigned to a class most common among *k* number of neighbors. In some cases, the distance-based classification model is selected from an LVQ classifier, a SOM classifier, a *k-*means clustering model, and a KNN classifier.

In embodiments where the distance-based classification model is a KNN classifier, *k* is a positive integer that may vary. In some embodiments, *k* ranges from 1 *to m,* where *m* is equal to half of the number of data points. In some cases, *k* ranges from 2 to 5. In certain cases, *k* is 1 or more, such as 2 or more, such as 3 or more, such as 4 or more, such as 5 or more, such as 6 or more, such as 7 or more, such as 8 or more, such as 9 or more, and including 10 or more. The method by which the *k* nearest neighbors is calculated may vary. In some cases, the method comprises calculating the *k* nearest neighbors using a vantage-point tree, a *k*-dimensional tree, ball tree, cover tree, locality-sensitive hashing, hierarchical navigable small world, approximate nearest neighbors with random projection trees, GPU-based KNN search, or a brute force KNN search. In some cases, the method comprises calculating the *k* nearest neighbors using a vantage-point tree. Vantage-point trees are described in, e.g., Yianilos, Peter N. Soda . (1993) 93(194):311-21, incorporated by reference herein. In select instances, the method comprises calculating the *k* nearest neighbors using a *k*-dimensional tree (*k*-d tree). *k*-dimensional trees are described in, e.g., Bentley, J. L. Comm. ACM. (1975) 18(9):509 517, incorporated by reference herein. In select instances, the method comprises calculating the *k* nearest neighbors using a ball tree (metric tree). Ball trees are described in, e.g., Omohundro, S. M. Five balltree construction algorithms. (1989)*,* incorporated by reference herein. In some cases, the method comprises calculating the *k* nearest neighbors using locally sensitive hashing. Locally sensitive hashing is described in, e.g., Paulevé et al. Pattern recognition letters. (2010) 31(11): 1348-1358, incorporated by reference herein. In some cases, the method comprises calculating the *k* nearest neighbors using hierarchical navigable small world (HNSW). Hierarchical navigable small world is described in, e.g., Malkov et al. IEEE transactions on pattern analysis and machine intelligence. (2018) 42(4): 824-836, incorporated by reference herein. In some cases, the method comprises calculating the k nearest neighbors using approximate nearest neighbors with random projection trees. Such trees are described in, e.g., Hyvönen et al. In 2016 IEEE International Conference on Big Data (Big Data), pp. 881-888, incorporated by reference herein. In some cases, the method comprises calculating the *k* nearest neighbors using a GPU-based KNN search. GPU-based KNN searches are described in, e.g., Garcia et al. In 2010 IEEE International Conference on Image Processing, pp. 3757-3760, incorporated by reference herein. In some cases, the method comprises performing a brute force KNN search.

While the use of other types of distance-based classifiers is envisioned (e.g., LVQ classifier, a SOM classifier, a *k*-means clustering model), the present inventor has realized that a KNN classifier may be of particular interest for classifying analyte data. For example, KNN is a simple and intuitive algorithm that is easy to understand and implement. Furthermore, KNN makes no underlying assumptions about the data's distribution. It was found that KNN can be very effective with smaller datasets, and exhibits versatility in feature types. For example, KNN can handle both numerical and categorical data. In addition, KNN is highly adaptable. It can adapt immediately as new training data is collected.

The present inventor has realized that combining a decision tree ensemble (e.g., random forest classification model) with a distance-based classification model ensures diversity in decision making and thereby improves the quality of the decisions. In other words, the decision tree ensemble (e.g., random forest) and distance-based classification model (e.g., KNN classifier) make decisions based on very different principles (ensemble of decision trees vs. distance-based neighbors), which introduces diversity in the decision-making process. It was also realized that combining the two models can lead to higher accuracy than either algorithm alone, especially if their individual errors are uncorrelated. Moreover, it was realized that the combination balances bias and variance. For example, random forest's method of reducing variance and KNN's low-bias characteristic can complement each other. This combination can furthermore handle different types of data and relationships. For example, Random Forest's strength in handling complex, non-linear relationships and KNN's effectiveness in capturing local similarities can be synergistic. It was also noted by the inventor that the combination has a robustness to noisy data. For example, the combination can be more robust to noise and outliers, as Random Forest can average out some of the noise, while KNN can adapt to changes in the data distribution.

Methods according to some embodiments of the disclosure also include training. In such embodiments, the subject classification models are provided with a training dataset. The training data may be received from any suitable source. In some embodiments, flow cytometer data is received from the memory of a storage device. In such embodiments, flow cytometer data may have been previously generated and saved in the memory of the storage device for subsequent recall and analysis. In embodiments, analyte data within the training dataset is of known classification. For example, in some cases where the training dataset includes flow cytometer data, each individual analyte may have been confirmed to correspond to one class or another by some other means. In certain instances, an expert user manually provides classifications to the training dataset. Such can include, e.g., manually drawing gates on a two-dimensional plot of flow cytometer data. Analyte features from the training dataset as well as these classifications may be provided for training purposes. In some embodiments, methods include training using a plurality of training datasets, such as 2 or more training datasets, such as 3 or more training datasets, such as 4 or more training datasets, and including 5 or more training datasets. In embodiments of the disclosure involving training, methods may include training the decision tree ensemble using analyte features from the training dataset. As discussed above, a result of running the analyte features through the decision tree ensemble is a set of predicted classes, e.g., in a column. These predicted classes are then provided to the distance-based classification model, which is subsequently trained on a combination of the predicted classes and the analyte features. Accordingly, in some implementations, the present disclosure may be conceptualized as training a model (e.g., decision tree ensemble) using a first dataset (e.g., comprising analyte features), generating a second dataset (e.g., comprising the analyte features and predicted classes), and training a model (e.g., distance-based classification model) using the second dataset.

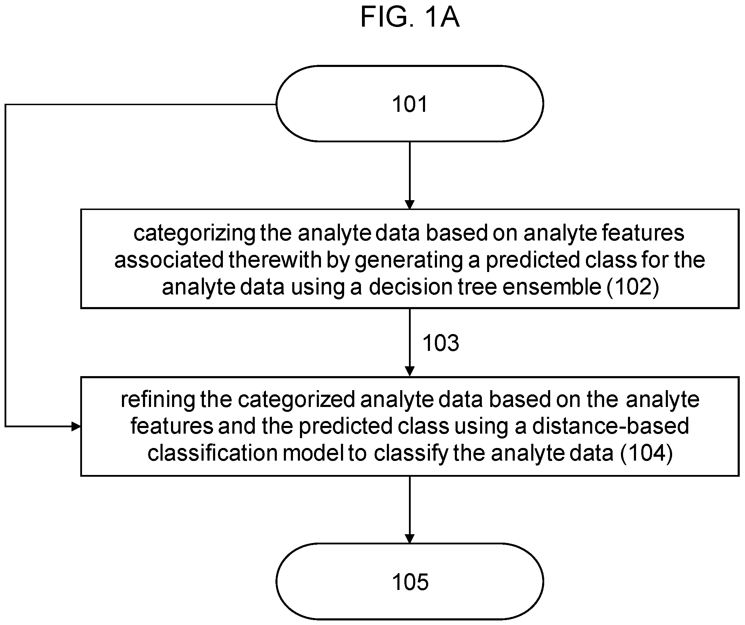

**FIG. 1A** presents a flow diagram for classifying analyte data according to one embodiment of the disclosure. As shown in **FIG. 1A****,** analyte data comprising analyte features 101 are received as an input. Step 102 includes categorizing the analyte data based on analyte features 101 associated therewith by generating a predicted class 103 for the analyte data using a decision tree ensemble. Step 104 includes refining the categorized analyte data based on the analyte features 101 and the predicted class 103 using a distance-based classification model. The result of step 104 is classified analyte data 105. Training the models would follow a corresponding process. In such a case, analyte features 101 would be from a training dataset and would be used to train the decision tree ensemble in step 102. Predicted classes 103 along with analyte features 101 from the training dataset would then be used to train the distance-based classification model in step 104.

In some embodiments, methods additionally include producing an image of the classified analyte data. Any suitable image may be produced. In some embodiments, methods include rendering the analyte data on a plot, such as a two-dimensional plot. Methods may include representing analyte data (e.g., events) differently based on how it is classified (e.g., as described above). For example, in some embodiments, methods include rendering a gate around the classified analyte data. For example, in some cases, single cells/singlets may be located within a first gate, doublets may be located within a second gate, and debris may be located within a third gate. Alternatively or in addition, methods may include representing different analyte data/events using different colors. For example, in some cases, single cells/singlets may be represented with a first color, doublets may be represented with a second color, and debris may be represented with a third color. However, any suitable method for depicting events with different classifications may be employed.

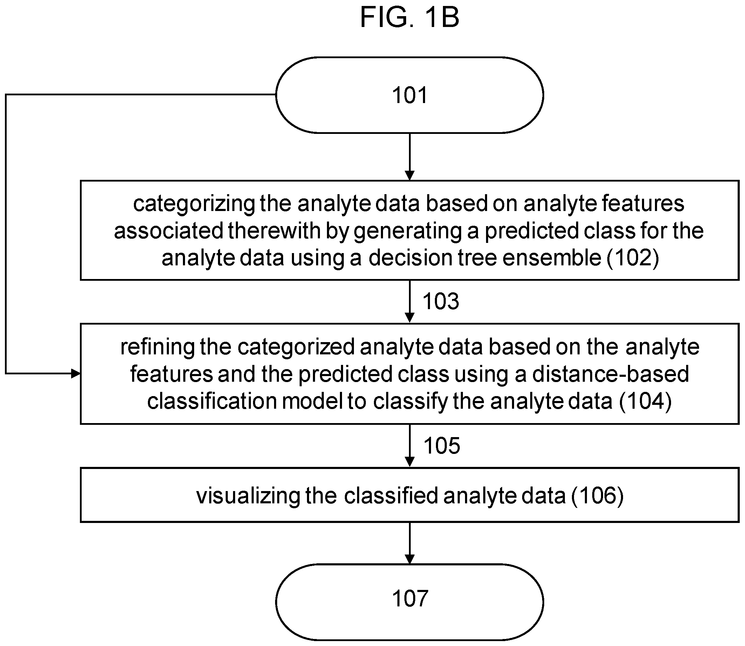

**FIG. 1B** presents a flow diagram for classifying analyte data that involves generating an image. **FIG. 1B** includes the same elements as **FIG. 1A** with the addition of visualizing the classified analyte data in step 106. Image 107 is subsequently outputted to the user.

Methods in certain embodiments also include data acquisition, analysis and recording, such as with a computer, wherein multiple data channels record data from each detector for the light scatter and fluorescence emitted by each particle as it passes through the sample interrogation region of the particle sorting module. In these embodiments, analysis includes classifying and counting particles such that each particle is present as a set of digitized parameter values. The subject systems may be set to trigger on a selected parameter in order to distinguish the particles of interest from background and noise. "Trigger" refers to a preset threshold for detection of a parameter and may be used as a means for detecting passage of a particle through the light source. Detection of an event that exceeds the threshold for the selected parameter triggers acquisition of light scatter and fluorescence data for the particle. Data is not acquired for particles or other components in the medium being assayed which cause a response below the threshold. The trigger parameter may be the detection of forward-scattered light caused by passage of a particle through the light beam. The flow cytometer then detects and collects the light scatter and fluorescence data for the particle. The data recorded for each particle is analyzed in real time or stored in a data storage and analysis means, such as a computer, as desired.

Methods of interest may additionally include sorting particles in a sample via a sorting flow cytometer based on the classification. Put another way, particles corresponding to flow cytometer data may be sorted into a series of collection vessels based on the status of classifications determined by the process described herein. For example, embodiments of the method include sorting particles associated with the set of flow cytometer data of a first classification into a first collection vessel, sorting particles associated with the set of flow cytometer data of a second classification into a second collection vessel, and so on. In certain instances, particles sorted may be considered "boundary" cases that cannot be neatly categorized but are likely to possess a sufficient number of particles of interest that it would be undesirable to discard them. Certain embodiments further include re-sorting the particles to obtain a higher yield of particles of interest.

Suitable collection vessels for collecting particles may include, but are not limited to: test tubes, conical tubes, multi-compartment vessels such as microtiter plates (e.g., 96-well plates), centrifuge tubes, culture tubes, microtubes, caps, cuvettes, bottles, rectilinear polymeric vessels, and bags, among other types of vessels. Particles may be sorted into any convenient number of collection vessels, such as 2 or more collection vessels, 3 or more collection vessels, 4 or more collection vessels, 5 or more collection vessels, 6 or more collection vessels, and including 7 or more collection vessels.

In some instances, the sample analyzed in the instant methods is a biological sample. The term "biological sample" is used in its conventional sense to refer to a whole organism, plant, fungi or a subset of animal tissues, cells or component parts which may in certain instances be found in blood, mucus, lymphatic fluid, synovial fluid, cerebrospinal fluid, saliva, bronchoalveolar lavage, amniotic fluid, amniotic cord blood, urine, vaginal fluid and semen. As such, a "biological sample" refers to both the native organism or a subset of its tissues as well as to a homogenate, lysate or extract prepared from the organism or a subset of its tissues, including but not limited to, for example, plasma, serum, spinal fluid, lymph fluid, sections of the skin, respiratory, gastrointestinal, cardiovascular, and genitourinary tracts, tears, saliva, milk, blood cells, tumors, organs. Biological samples may be any type of organismic tissue, including both healthy and diseased tissue (e.g., cancerous, malignant, necrotic, etc.). In certain embodiments, the biological sample is a liquid sample, such as blood or derivative thereof, e.g., plasma, tears, urine, semen, etc., where in some instances the sample is a blood sample, including whole blood, such as blood obtained from venipuncture or fingerstick (where the blood may or may not be combined with any reagents prior to assay, such as preservatives, anticoagulants, etc.).

In certain embodiments the source of the sample is a "mammal" or "mammalian", where these terms are used broadly to describe organisms which are within the class Mammalia, including the orders carnivore (e.g., dogs and cats), Rodentia (e.g., mice, guinea pigs, and rats), and primates (e.g., humans, chimpanzees, and monkeys). In some instances, the subjects are humans. The methods may be applied to samples obtained from human subjects of both genders and at any stage of development (i.e., neonates, infant, juvenile, adolescent, adult), where in certain embodiments the human subject is a juvenile, adolescent or adult. While the present disclosure may be applied to samples from a human subject, it is to be understood that the methods may also be carried-out on samples from other animal subjects (that is, in "non-human subjects") such as, but not limited to, birds, mice, rats, dogs, cats, livestock and horses.

Cells of interest may be targeted for characterized according to a variety of parameters, such as a phenotypic characteristic identified via the attachment of a particular fluorescent label to cells of interest. In some embodiments, the system is configured to deflect analyzed droplets that are determined to include a target cell. A variety of cells may be characterized using the subject methods. Target cells of interest include, but are not limited to, stem cells, T cells, dendritic cells, B Cells, granulocytes, leukemia cells, lymphoma cells, virus cells (e.g., HIV cells), NK cells, macrophages, monocytes, fibroblasts, epithelial cells, endothelial cells, and erythroid cells. Target cells of interest include cells that have a convenient cell surface marker or antigen that may be captured or labelled by a convenient affinity agent or conjugate thereof. For example, the target cell may include a cell surface antigen such as CD11b, CD123, CD14, CD15, CD16, CD19, CD193, CD2, CD25, CD27, CD3, CD335, CD36, CD4, CD43, CD45RO, CD56, CD61, CD7, CD8, CD34, CD1c, CD23, CD304, CD235a, T cell receptor alpha/beta, T cell receptor gamma/delta, CD253, CD95, CD20, CD105, CD117, CD120b, Notch4, Lgr5 (N-Terminal), SSEA-3, TRA-1-60 Antigen, Disialoganglioside GD2 and CD71. In some embodiments, the target cell is selected from HIV containing cell, a Treg cell, an antigen-specific T -cell populations, tumor cells or hematopoietic progenitor cells (CD34+) from whole blood, bone marrow or cord blood.

Methods of interest may further include employing particles in research, laboratory testing, or therapy. In some embodiments, the subject methods include obtaining individual cells prepared from a target fluidic or tissue biological sample. For example, the subject methods include obtaining cells from fluidic or tissue samples to be used as a research or diagnostic specimen for diseases such as cancer. Likewise, the subject methods include obtaining cells from fluidic or tissue samples to be used in therapy. A cell therapy protocol is a protocol in which viable cellular material including, e.g., cells and tissues, may be prepared and introduced into a subject as a therapeutic treatment. Conditions that may be treated by the administration of the flow cytometrically sorted sample include, but are not limited to, blood disorders, immune system disorders, organ damage, etc.

A typical cell therapy protocol may include the following steps: sample collection, cell isolation, genetic modification, culture, and expansion in vitro, cell harvesting, sample volume reduction and washing, bio-preservation, storage, and introduction of cells into a subject. The protocol may begin with the collection of viable cells and tissues from source tissues of a subject to produce a sample of cells and/or tissues. The sample may be collected via any suitable procedure that includes, e.g., administering a cell mobilizing agent to a subject, drawing blood from a subject, removing bone marrow from a subject, etc. After collecting the sample, cell enrichment may occur via several methods including, e.g., centrifugation based methods, filter based methods, elutriation, magnetic separation methods, fluorescence-activated cell sorting (FACS), and the like. In some cases, the enriched cells may be genetically modified by any convenient method, e.g., nuclease mediated gene editing. The genetically modified cells can be cultured, activated, and expanded in vitro. In some cases, the cells are preserved, e.g., cryopreserved, and stored for future use where the cells are thawed and then administered to a patient, e.g., the cells may be infused in the patient.

### SYSTEMS

Aspects of the disclosure also include systems for classifying analyte data. Systems of interest include memory operably coupled to a processor, which when executed by the processor, cause the processor to carry out the methods of the disclosure. As discussed above, such methods include categorizing analyte data based on analyte features associated therewith by generating a predicted class for the analyte data using a decision tree ensemble, and refining the categorized analyte data based on the analyte features and the predicted class using a distance-based classification model to classify the analyte data.

Systems may include a display and operator input device. Operator input devices may, for example, be a keyboard, mouse, or the like. The processing module includes a processor which has access to a memory having instructions stored thereon for performing the steps of the subject methods. The processing module may include an operating system, a graphical user interface (GUI) controller, a system memory, memory storage devices, and input-output controllers, cache memory, a data backup unit, and many other devices. The processor may be a commercially available processor, or it may be one of other processors that are or will become available. The processor executes the operating system and the operating system interfaces with firmware and hardware in a well-known manner, and facilitates the processor in coordinating and executing the functions of various computer programs that may be written in a variety of programming languages, such as Java, Perl, C++, Python, other high level or low level languages, as well as combinations thereof, as is known in the art. The operating system, typically in cooperation with the processor, coordinates and executes functions of the other components of the computer. The operating system also provides scheduling, input-output control, file and data management, memory management, and communication control and related services, all in accordance with known techniques. In some embodiments, the processor includes analog electronics which provide feedback control, such as for example negative feedback control.

The system memory may be any of a variety of known or future memory storage devices. Examples include any commonly available random access memory (RAM), magnetic medium such as a resident hard disk or tape, an optical medium such as a read and write compact disc, flash memory devices, or other memory storage device. The memory storage device may be any of a variety of known or future devices, including a compact disk drive, a tape drive, or a diskette drive. Such types of memory storage devices typically read from, and/or write to, a program storage medium (not shown) such as a compact disk. Any of these program storage media, or others now in use or that may later be developed, may be considered a computer program product. As will be appreciated, these program storage media typically store a computer software program and/or data. Computer software programs, also called computer control logic, typically are stored in system memory and/or the program storage device used in conjunction with the memory storage device.

In some embodiments, a computer program product is described comprising a computer usable medium having control logic (computer software program, including program code) stored therein. The control logic, when executed by the processor the computer, causes the processor to perform functions described herein. In other embodiments, some functions are implemented primarily in hardware using, for example, a hardware state machine. Implementation of the hardware state machine so as to perform the functions described herein will be apparent to those skilled in the relevant arts.

The subject programmable logic may be implemented in any of a variety of devices such as specifically programmed event processing computers, wireless communication devices, integrated circuit devices, or the like. In some embodiments, the programable logic may be executed by a specifically programmed processor, which may include one or more processors, such as one or more digital signal processors (DSPs), configurable microprocessors, application specific integrated circuits (ASICs), field programmable gate arrays (FPGAs), or other equivalent integrated or discrete logic circuitry. A combination of computing devices, e.g., a combination of a DSP and a microprocessor, a plurality of microprocessors, one or more microprocessors in conjunction with a DSP core, or any other such configuration in at least partial data connectivity may implement one or more of the features described herein.

Memory may be any suitable device in which the processor can store and retrieve data, such as magnetic, optical, or solid-state storage devices (including magnetic or optical disks or tape or RAM, or any other suitable device, either fixed or portable). The processor may include a general-purpose digital microprocessor suitably programmed from a computer readable medium carrying necessary program code. Programming can be provided remotely to processor through a communication channel, or previously saved in a computer program product such as memory or some other portable or fixed computer readable storage medium using any of those devices in connection with memory. For example, a magnetic or optical disk may carry the programming, and can be read by a disk writer/reader. Systems of the disclosure also include programming, e.g., in the form of computer program products, algorithms for use in practicing the methods as described above. Programming according to the present disclosure can be recorded on computer readable media, e.g., any medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; portable flash drive; and hybrids of these categories such as magnetic/optical storage media.

The processor may also have access to a communication channel to communicate with a user at a remote location. By remote location is meant the user is not directly in contact with the system and relays input information to an input manager from an external device, such as a computer connected to a Wide Area Network ("WAN"), telephone network, satellite network, or any other suitable communication channel, including a mobile telephone (i.e., smartphone).

In some embodiments, systems according to the present disclosure may be configured to include a communication interface. In some embodiments, the communication interface includes a receiver and/or transmitter for communicating with a network and/or another device. The communication interface can be configured for wired or wireless communication, including, but not limited to, radio frequency (RF) communication (e.g., Radio-Frequency Identification (RFID), Zigbee communication protocols, Wi-Fi, infrared, wireless Universal Serial Bus (USB), Ultra Wide Band (UWB), Bluetooth^{®} communication protocols, and cellular communication, such as code division multiple access (CDMA) or Global System for Mobile communications (GSM).

In one embodiment, the communication interface is configured to include one or more communication ports, e.g., physical ports or interfaces such as a USB port, a USB-C port, an RS-232 port, or any other suitable electrical connection port to allow data communication between the subject systems and other external devices such as a computer terminal (for example, at a physician's office or in hospital environment) that is configured for similar complementary data communication.

In one embodiment, the communication interface is configured for infrared communication, Bluetooth^{®} communication, or any other suitable wireless communication protocol to enable the subject systems to communicate with other devices such as computer terminals and/or networks, communication enabled mobile telephones, personal digital assistants, or any other communication devices which the user may use in conjunction.

In one embodiment, the communication interface is configured to provide a connection for data transfer utilizing Internet Protocol (IP) through a cell phone network, Short Message Service (SMS), wireless connection to a personal computer (PC) on a Local Area Network (LAN) which is connected to the internet, or Wi-Fi connection to the internet at a Wi-Fi hotspot.

In one embodiment, the subject systems are configured to wirelessly communicate with a server device via the communication interface, e.g., using a common standard such as 802.11 or Bluetooth^{®} RF protocol, or an IrDA infrared protocol. The server device may be another portable device, such as a smart phone, Personal Digital Assistant (PDA) or notebook computer; or a larger device such as a desktop computer, appliance, etc. In some embodiments, the server device has a display, such as a liquid crystal display (LCD), as well as an input device, such as buttons, a keyboard, mouse or touch-screen.

In some embodiments, the communication interface is configured to automatically or semi-automatically communicate data stored in the subject systems, e.g., in an optional data storage unit, with a network or server device using one or more of the communication protocols and/or mechanisms described above.

Output controllers may include controllers for any of a variety of known display devices for presenting information to a user, whether a human or a machine, whether local or remote. If one of the display devices provides visual information, this information typically may be logically and/or physically organized as an array of picture elements. A graphical user interface (GUI) controller may include any of a variety of known or future software programs for providing graphical input and output interfaces between the system and a user, and for processing user inputs. The functional elements of the computer may communicate with each other via system bus. Some of these communications may be accomplished in alternative embodiments using network or other types of remote communications. The output manager may also provide information generated by the processing module to a user at a remote location, e.g., over the Internet, phone or satellite network, in accordance with known techniques. The presentation of data by the output manager may be implemented in accordance with a variety of known techniques. As some examples, data may include SQL, HTML or XML documents, email or other files, or data in other forms. The data may include Internet URL addresses so that a user may retrieve additional SQL, HTML, XML, or other documents or data from remote sources. The one or more platforms present in the subject systems may be any type of known computer platform or a type to be developed in the future, although they typically will be of a class of computer commonly referred to as servers. However, they may also be a main-frame computer, a workstation, or other computer type. They may be connected via any known or future type of cabling or other communication system including wireless systems, either networked or otherwise. They may be co-located or they may be physically separated. Various operating systems may be employed on any of the computer platforms, possibly depending on the type and/or make of computer platform chosen. Appropriate operating systems include Windows^{®} NT^{®}, Windows^{®} XP, Windows^{®} 7, Windows^{®} 8, Windows^{®} 10, iOS^{®}, macOS^{®}, Linux^{®}, Ubuntu^{®}, Fedora^{®}, OS/400^{®}, i5/OS^{®}, IBM i^{®}, Android^{™}, SGI IRIX^{®}, Oracle Solaris^{®} and others.

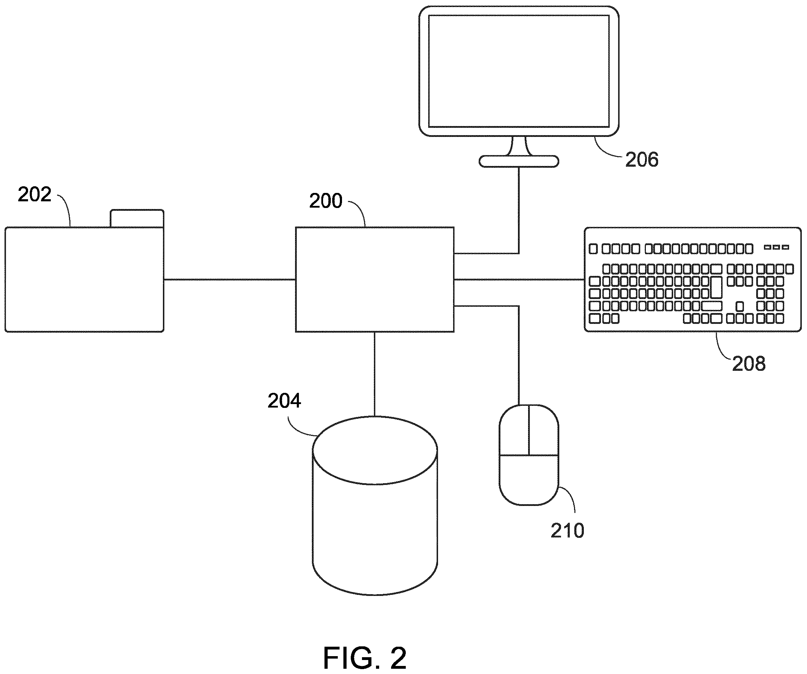

**FIG. 2** shows a functional block diagram for one example of a system for classifying analyte data according to certain embodiments. As shown in **FIG. 2****,** a processor 200 can be configured to implement a variety of processes for classifying analyte data by categorizing the analyte data based on analyte features associated therewith by generating a predicted class for the analyte data using a decision tree ensemble, and refining the categorized analyte data based on the analyte features and the predicted class using a distance-based classification model to classify the analyte data.

A flow cytometer or sorting system 202 can be configured to acquire biological event data. For example, a flow cytometer can generate flow cytometric event data (e.g., particle-modulated light data). The flow cytometer 202 can be configured to provide biological event data to the processor 200. A data communication channel can be included between the flow cytometer 202 and the processor 200. The biological event data can be provided to the processor 200 via the data communication channel.

The processor 200 can be configured to receive biological event data from the flow cytometer 202. The biological event data received from the flow cytometer 202 can include flow cytometric event data. The processor 200 can be further configured to display the biological event data on the display device 206 within one classification differently from other events in the biological event data outside of the gate. For example, the processor 200 can be configured to render the color of biological event data contained within one classification to be distinct from the color of biological event data of another classification. Alternatively, the processor 200 can be configured to render gates around flow cytometer data of different classifications. The display device 206 can be implemented as a monitor, a tablet computer, a smartphone, or other electronic device configured to present graphical interfaces.

The processor 200 can be configured to receive a gate selection signal identifying the gate from a first input device. For example, the first input device can be implemented as a mouse 210. The mouse 210 can initiate a gate selection signal to the processor 200 identifying the gate to be displayed on or manipulated via the display device 206 (e.g., by clicking on or in the desired gate when the cursor is positioned there). In some implementations, the first device can be implemented as the keyboard 208 or other means for providing an input signal to the processor 200 such as a touchscreen, a stylus, an optical detector, or a voice recognition system. Some input devices can include multiple inputting functions. In such implementations, the inputting functions can each be considered an input device. For example, as shown in **FIG. 2****,** the mouse 210 can include a right mouse button and a left mouse button, each of which can generate a triggering event. The triggering event can cause the processor 200 to alter the manner in which the data is displayed, which portions of the data is actually displayed on the display device 206, and/or provide input to further processing such as selection of a population of interest for particle sorting.

In some embodiments, the processor 200 can be configured to detect when gate selection is initiated by the mouse 210. The processor 200 can be further configured to automatically modify plot visualization to facilitate the gating process. The modification can be based on the specific distribution of biological event data received by the processor 200.

The processor 200 can be connected to a storage device 204. The storage device 204 can be configured to receive and store biological event data from the processor 200. The storage device 204 can also be configured to receive and store flow cytometric event data from the processor 200. The storage device 204 can be further configured to allow retrieval of biological event data, such as flow cytometric event data, by the processor 200.

The display device 206 can be configured to receive display data from the processor 200. The display data can comprise plots of biological event data and gates outlining sections of the plots. The display device 206 can be further configured to alter the information presented according to input received from the processor 200 in conjunction with input from the flow cytometer 202, the storage device 204, the keyboard 208, and/or the mouse 210.

In some implementations the processor 200 can generate a user interface to receive example events for sorting. For example, the user interface can include a mechanism for receiving example events or example images. The example events or images or an example gate can be provided prior to collection of event data for a sample or based on an initial set of events for a portion of the sample.

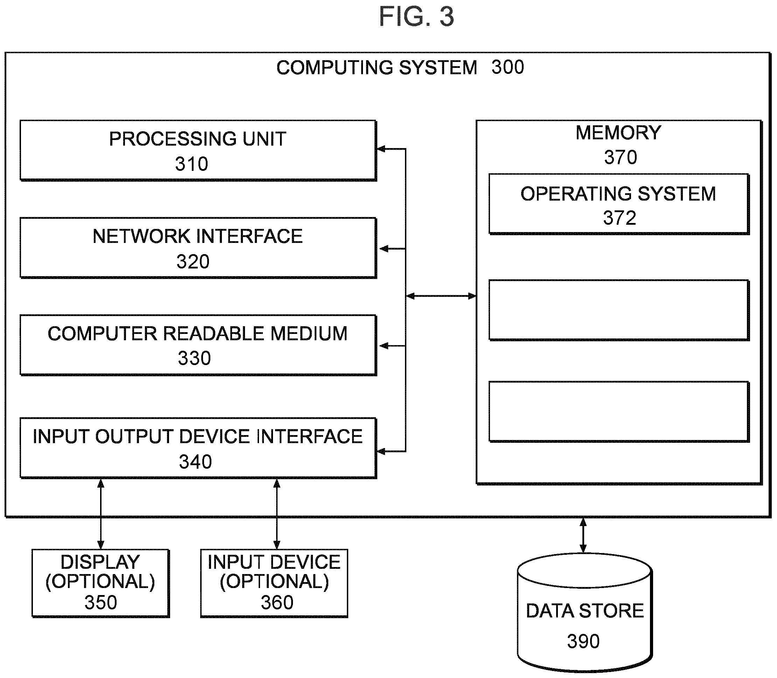

**FIG. 3** depicts a general architecture of an example computing device 300 according to certain embodiments. The general architecture of the computing device 300 depicted in **FIG. 3** includes an arrangement of computer hardware and software components. It is not necessary, however, that all of these generally conventional elements be shown in order to provide an enabling disclosure. As illustrated, the computing device 300 includes a processing unit 310, a network interface 320, a computer readable medium drive 330, an input/output device interface 340, a display 350, and an input device 360, all of which may communicate with one another by way of a communication bus. The network interface 320 may provide connectivity to one or more networks or computing systems. The processing unit 310 may thus receive information and instructions from other computing systems or services via a network. The processing unit 310 may also communicate to and from memory 370 and further provide output information for an optional display 350 via the input/output device interface 340. For example, an analysis software (e.g., data analysis software or program such as FlowJo^{®}) stored as executable instructions in the non-transitory memory of the analysis system can display the flow cytometry event data to a user. The input/output device interface 340 may also accept input from the optional input device 360, such as a keyboard, mouse, digital pen, microphone, touch screen, gesture recognition system, voice recognition system, gamepad, accelerometer, gyroscope, or other input device.

The memory 370 may contain computer program instructions (grouped as modules or components in some embodiments) that the processing unit 310 executes in order to implement one or more embodiments. The memory 370 generally includes RAM, ROM and/or other persistent, auxiliary or non-transitory computer-readable media. The memory 370 may store an operating system 372 that provides computer program instructions for use by the processing unit 310 in the general administration and operation of the computing device 300. Data may be stored in data storage device 390. The memory 370 may further include computer program instructions and other information for implementing aspects of the present disclosure.

In some embodiments, the system comprises a flow cytometer. Flow cytometers of interest include a flow cell. The term "flow cell" is described in its conventional sense to refer to a component, such as a cuvette, containing a flow channel having a liquid flow stream for transporting particles in a sheath fluid. Cuvettes of interest include containers having a passage running therethrough. The flow stream may include a liquid sample injected from a sample tube. Flow cells of interest include a light-accessible flow channel. In some instances, the flow cell includes transparent material (e.g., quartz) that permits the passage of light therethrough. In some embodiments, the flow cell is a stream-in-air flow cell in which light interrogation of the particles occurs in free space. In some cases, the flow stream is configured for irradiation with light from one or more light sources at interrogation points. As discussed herein, an "interrogation point" refers to a region within the flow stream in which a particle is irradiated by light from a light source, e.g., for analysis. The size of the interrogation point may vary as desired. For example, where 0 µm represents the axis of light emitted by the light relevant light source, the interrogation zone may range from -100 µm to 100 µm, such as -50 µm to 50 µm, such as -25 µm to 40 µm, and including -15 µm to 30 µm. The flow stream for which the flow channel is configured may include a liquid sample injected from a sample tube. In certain embodiments, the flow stream may include a narrow, rapidly flowing stream of liquid that is arranged such that linearly segregated particles transported therein are separated from each other in a single-file manner. After particles are irradiated in the flow cell, particle-modulated light may be observed.

Any convenient flow cell which propagates a fluidic sample to a sample interrogation region may be employed, where in some embodiments, the flow cell includes is a cylindrical flow cell, a frustoconical flow cell or a flow cell that includes a proximal cylindrical portion defining a longitudinal axis and a distal frustoconical portion which terminates in a flat surface having the orifice that is transverse to the longitudinal axis.

In some embodiments, the sample flow stream emanates from an orifice at the distal end of the flow cell. Depending on the desired characteristics of the flow stream, the flow cell orifice may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. In certain embodiments, flow cell of interest has a circular orifice. The size of the nozzle orifice may vary, in some embodiments ranging from 1 µm to 10000 µm, such as from 25 µm to 7500 µm, such as from 50 µm to 5000 µm, such as from 75 µm to 1000 µm, such as from 100 µm to 750 µm and including from 150 µm to 500 µm. In certain embodiments, the nozzle orifice is 100 µm.

In some embodiments, the flow cell includes a sample injection port configured to provide a sample to the flow cell. The sample injection port may be an orifice positioned in a wall of the inner chamber or may be a conduit positioned at the proximal end of the inner chamber. Where the sample injection port is an orifice positioned in a wall of the inner chamber, the sample injection port orifice may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, etc., as well as irregular shapes, e.g., a parabolic bottom portion coupled to a planar top portion. In certain embodiments, the sample injection port has a circular orifice. The size of the sample injection port orifice may vary depending on shape, in certain instances, having an opening ranging from 0.1 mm to 5.0 mm, such as 0.2 to 3.0 mm, such as 0.5 mm to 2.5 mm, such as from 0.75 mm to 2.25 mm, such as from 1 mm to 2 mm and including from 1.25 mm to 1.75 mm, for example 1.5 mm.

In certain instances, the sample injection port is a conduit positioned at a proximal end of the flow cell inner chamber. For example, the sample injection port may be a conduit positioned to have the orifice of the sample injection port in line with the flow cell orifice. Where the sample injection port is a conduit positioned in line with the flow cell orifice, the cross-sectional shape of the sample injection tube may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The orifice of the conduit may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm. The shape of the tip of the sample injection port may be the same or different from the cross-sectional shape of the sample injection tube. For example, the orifice of the sample injection port may include a beveled tip having a bevel angle ranging from 1 degree to 10 degrees, such as from 2 degrees to 9 degrees, such as from 3 degrees to 8 degrees, such as from 4 degrees to 7 degrees and including a bevel angle of 5 degrees.

In some embodiments, the flow cell also includes a sheath fluid injection port configured to provide a sheath fluid to the flow cell. In embodiments, the sheath fluid injection system is configured to provide a flow of sheath fluid to the flow cell inner chamber, for example in conjunction with the sample to produce a laminated flow stream of sheath fluid surrounding the sample flow stream. Depending on the desired characteristics of the flow stream, the rate of sheath fluid conveyed to the flow cell chamber may be 25 µL/sec to 2500 µL/sec, such as 50 µL/sec to 1000 µL/sec, and including 75 µL/sec or more to 750 µL/sec.

In some embodiments, the sheath fluid injection port is an orifice positioned in a wall of the inner chamber. The sheath fluid injection port orifice may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The size of the sample injection port orifice may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm.

In some embodiments, systems further include a pump in fluid communication with the flow cell to propagate the flow stream through the flow cell. Any convenient fluid pump protocol may be employed to control the flow of the flow stream through the flow cell. In certain instances, systems include a peristaltic pump, such as a peristaltic pump having a pulse damper. The pump in the subject systems is configured to convey fluid through the flow cell at a rate suitable for multi-photon counting of light from the sample in the flow stream. For example, the system may include a pump that is configured to flow sample through the flow cell at a rate that ranges from 1 nL/min to 500 nL/min, such as from 1 nL/min to 250 nL/min, such as from 1 nL/min to 100 nL/min, such as from 2 nL/min to 90 nL/min, such as from 3 nL/min to 80 nL/min, such as from 4 nL/min to 70 nL/min, such as from 5 nL/min to 60 nL/min and including from 10 nL/min to 50 nL/min. In certain embodiments, the flow rate of the flow stream is from 5 nL/min to 6 nL/min.

Aspects of the subject systems also includes a light source for irradiating the flow stream at an interrogation point. Any convenient light source may be employed, such as a laser. In embodiments, the laser may be any convenient laser, such as a continuous wave laser. For example, the laser may be a diode laser, such as an ultraviolet diode laser, a visible diode laser and a near-infrared diode laser. In other embodiments, the laser may be a helium-neon (HeNe) laser. In some instances, the laser is a gas laser, such as a helium-neon laser, argon laser, krypton laser, xenon laser, nitrogen laser, CO₂ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof. In other instances, the subject flow cytometers include a dye laser, such as a stilbene, coumarin or rhodamine laser. In yet other instances, lasers of interest include a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof. In still other instances, the subject flow cytometers include a solid-state laser, such as a ruby laser, an Nd:YAG laser, NdCrYAG laser, Er:YAG laser, Nd:YLF laser, Nd:YVO₄ laser, Nd:YCa₄O(BO₃)₃ laser, Nd:YCOB laser, titanium sapphire laser, thulim YAG laser, ytterbium YAG laser, ytterbium₂O₃ laser or cerium doped lasers and combinations thereof.

Laser light sources according to certain embodiments may also include one or more optical adjustment components. In certain embodiments, the optical adjustment component is located between the light source and the flow cell, and may include any device that is capable of changing the spatial width of irradiation or some other characteristic of irradiation from the light source, such as for example, irradiation direction, wavelength, beam width, beam intensity and focal spot. Optical adjustment protocols may include any convenient device which adjusts one or more characteristics of the light source, including but not limited to lenses, mirrors, filters, fiber optics, wavelength separators, pinholes, slits, collimating protocols and combinations thereof. In certain embodiments, flow cytometers of interest include one or more focusing lenses. The focusing lens, in one example, may be a de-magnifying lens. In still other embodiments, flow cytometers of interest include fiber optics.

Where the optical adjustment component is configured to move, the optical adjustment component may be configured to be moved continuously or in discrete intervals, such as for example in 0.01 µm or greater increments, such as 0.05 µm or greater, such as 0.1 µm or greater, such as 0.5 µm or greater such as 1 µm or greater, such as 10 µm or greater, such as 100 µm or greater, such as 500 µm or greater, such as 1 mm or greater, such as 5 mm or greater, such as 10 mm or greater and including 25 mm or greater increments.

Any displacement protocol may be employed to move the optical adjustment component structures, such as coupled to a moveable support stage or directly with a motor actuated translation stage, leadscrew translation assembly, geared translation device, such as those employing a stepper motor, servo motor, brushless electric motor, brushed DC motor, micro-step drive motor, high resolution stepper motor, among other types of motors.

The light source may be positioned any suitable distance from the flow cell, such as where the light source and the flow cell are separated by 0.005 mm or more, such as 0.01 mm or more, such as 0.05 mm or more, such as 0.1 mm or more, such as 0.5 mm or more, such as 1 mm or more, such as 5 mm or more, such as 10 mm or more, such as 25 mm or more and including at a distance of 100 mm or more. In addition, the light source may be positioned at any suitable angle relative to the flow cell, such as at an angle ranging from 10 degrees to 90 degrees, such as from 15 degrees to 85 degrees, such as from 20 degrees to 80 degrees, such as from 25 degrees to 75 degrees and including from 30 degrees to 60 degrees, for example at a 90 degree angle.

Systems according to certain embodiments include a plurality of light sources. In some cases, the plurality of light sources includes a plurality of lasers. such as 2 lasers or more, such as 3 lasers or more, such as 4 lasers or more, such as 5 lasers or more, such as 10 lasers or more, and including 15 lasers or more configured to provide laser light for discrete irradiation of the flow stream. Depending on the desired wavelengths of light for irradiating the flow stream, each laser may have a specific wavelength that varies from 200 nm to 1500 nm, such as from 250 nm to 1250 nm, such as from 300 nm to 1000 nm, such as from 350 nm to 900 nm and including from 400 nm to 800 nm. In certain embodiments, lasers of interest may include one or more of a 405 nm laser, a 488 nm laser, a 561 nm laser and a 635 nm laser.

Systems of interest may include one or more detectors for detecting particle-modulated light intensity data. In some embodiments, the particle-modulated light detector(s) include one or more forward-scattered light detectors configured to detect forward-scattered light. For example, the subject particle analyzers may include 1 forward-scattered light detector or multiple forward-scattered light detectors, such as 2 or more, such as 3 or more, such as 4 or more, and including 5 or more. In certain embodiments, particle analyzers include 1 forward-scattered light detector. In other embodiments, particle analyzers include 2 forward-scattered light detectors.

Any convenient detector for detecting collected light may be used in the forward-scattered light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. In certain embodiments, the collected light is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. In certain embodiments, the detector is a photomultiplier tube, such as a photomultiplier tube having an active detecting surface area of each region that ranges from 0.01 cm² to 10 cm², such as from 0.05 cm² to 9 cm², such as from 0.1 cm² to 8 cm², such as from 0.5 cm² to 7 cm² and including from 1 cm² to 5 cm².

In embodiments, the forward-scattered light detector is configured to measure light continuously or in discrete intervals. In some instances, detectors of interest are configured to take measurements of the collected light continuously. In other instances, detectors of interest are configured to take measurements in discrete intervals, such as measuring light every 0.001 millisecond, every 0.01 millisecond, every 0.1 millisecond, every 1 millisecond, every 10 milliseconds, every 100 milliseconds and including every 1000 milliseconds, or some other interval.

In some instances, systems include one or more side-scattered light detectors for detecting side-scatter wavelengths of light (i.e., light refracted and reflected from the surfaces and internal structures of the particle). In some embodiments, particle analyzers include a single side-scattered light detector. In other embodiments, particle analyzers include multiple side-scattered light detectors, such as 2 or more, such as 3 or more, such as 4 or more, and including 5 or more.

Any convenient detector for detecting collected light may be used in the side-scattered light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. In certain embodiments, the collected light is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. In certain embodiments, the detector is a photomultiplier tube, such as a photomultiplier tube having an active detecting surface area of each region that ranges from 0.01 cm² to 10 cm², such as from 0.05 cm² to 9 cm², such as from 0.1 cm² to 8 cm², such as from 0.5 cm² to 7 cm² and including from 1 cm² to 5 cm².

In embodiments, the subject systems also include a fluorescent light detector configured to detect one or more fluorescent wavelengths of light. In other embodiments, particle analyzers include multiple fluorescent light detectors such as 2 or more, such as 3 or more, such as 4 or more, 5 or more, 10 or more, 15 or more, and including 20 or more.

Any convenient detector for detecting collected light may be used in the fluorescent light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. In certain embodiments, the collected light is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. In certain embodiments, the detector is a photomultiplier tube, such as a photomultiplier tube having an active detecting surface area of each region that ranges from 0.01 cm² to 10 cm², such as from 0.05 cm² to 9 cm², such as from, such as from 0.1 cm² to 8 cm², such as from 0.5 cm² to 7 cm² and including from 1 cm² to 5 cm².

Where the subject particle analyzers include multiple fluorescent light detectors, each fluorescent light detector may be the same, or the collection of fluorescent light detectors may be a combination of different types of detectors. For example, where the subject particle analyzers include two fluorescent light detectors, in some embodiments the first fluorescent light detector is a CCD-type device and the second fluorescent light detector (or imaging sensor) is a CMOS-type device. In other embodiments, both the first and second fluorescent light detectors are CCD-type devices. In yet other embodiments, both the first and second fluorescent light detectors are CMOS-type devices. In still other embodiments, the first fluorescent light detector is a CCD-type device and the second fluorescent light detector is a photomultiplier tube (PMT). In still other embodiments, the first fluorescent light detector is a CMOS-type device and the second fluorescent light detector is a photomultiplier tube. In yet other embodiments, both the first and second fluorescent light detectors are photomultiplier tubes.

In embodiments of the present disclosure, fluorescent light detectors of interest are configured to measure collected light at one or more wavelengths, such as at 2 or more wavelengths, such as at 5 or more different wavelengths, such as at 10 or more different wavelengths, such as at 25 or more different wavelengths, such as at 50 or more different wavelengths, such as at 100 or more different wavelengths, such as at 200 or more different wavelengths, such as at 300 or more different wavelengths and including measuring light emitted by a sample in the flow stream at 400 or more different wavelengths. In some embodiments, 2 or more detectors in the particle analyzers as described herein are configured to measure the same or overlapping wavelengths of collected light.

In some embodiments, fluorescent light detectors of interest are configured to measure collected light over a range of wavelengths (e.g., 200 nm - 1000 nm). In certain embodiments, detectors of interest are configured to collect spectra of light over a range of wavelengths. For example, particle analyzers may include one or more detectors configured to collect spectra of light over one or more of the wavelength ranges of 200 nm - 1000 nm. In yet other embodiments, detectors of interest are configured to measure light emitted by a sample in the flow stream at one or more specific wavelengths. For example, particle analyzers may include one or more detectors configured to measure light at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof. In certain embodiments, one or more detectors may be configured to be paired with specific fluorophores, such as those used with the sample in a fluorescence assay.

In some embodiments, one or more of the particle-modulated light detectors includes one or more detector arrays, such as an array of photodiodes. In these embodiments, each detector array may include 4 or more detectors, such as 10 or more detectors, such as 25 or more detectors, such as 50 or more detectors, such as 100 or more detectors, such as 250 or more detectors, such as 500 or more detectors, such as 750 or more detectors and including 1000 or more detectors. For example, the detector may be a photodiode array having 4 or more photodiodes, such as 10 or more photodiodes, such as 25 or more photodiodes, such as 50 or more photodiodes, such as 100 or more photodiodes, such as 250 or more photodiodes, such as 500 or more photodiodes, such as 750 or more photodiodes and including 1000 or more photodiodes.

The detectors may be arranged in any geometric configuration as desired, where arrangements of interest include, but are not limited to a square configuration, rectangular configuration, trapezoidal configuration, triangular configuration, hexagonal configuration, heptagonal configuration, octagonal configuration, nonagonal configuration, decagonal configuration, dodecagonal configuration, circular configuration, oval configuration as well as irregular patterned configurations. The detectors in the detector array may be oriented with respect to the other (as referenced in an X-Z plane) at an angle ranging from 10° to 180°, such as from 15° to 170°, such as from 20° to 160°, such as from 25° to 150°, such as from 30° to 120° and including from 45° to 90°. The detector array may be any suitable shape and may be a rectilinear shape, e.g., squares, rectangles, trapezoids, triangles, hexagons, etc., curvilinear shapes, e.g., circles, ovals, as well as irregular shapes, e.g., a parabolic bottom portion coupled to a planar top portion. In certain embodiments, the detector array has a rectangular-shaped active surface.

In some embodiments, particle analyzers include one or more wavelength separators positioned between the flow cell and the particle-modulated light detector(s). The term "wavelength separator" is used herein in its conventional sense to refer to an optical component that is configured to separate light collected from the sample into predetermined spectral ranges. In some embodiments, particle analyzers include a single wavelength separator. In other embodiments, particle analyzers include a plurality of wavelength separators, such as 2 or more wavelength separators, such as 3 or more, such as 4 or more, such as 5 or more, such as 6 or more, such as 7 or more, such as 8 or more, such as 9 or more, such as 10 or more, such as 15 or more, such as 25 or more, such as 50 or more, such as 75 or more and including 100 or more wavelength separators. In some embodiments, the wavelength separator is configured to separate light collected from the sample into predetermined spectral ranges by passing light having a predetermined spectral range and reflecting one or more remaining spectral ranges of light. In other embodiments, the wavelength separator is configured to separate light collected from the sample into predetermined spectral ranges by passing light having a predetermined spectral range and absorbing one or more remaining spectral ranges of light. In yet other embodiments, the wavelength separator is configured to spatially diffract light collected from the sample into predetermined spectral ranges. Each wavelength separator may be any convenient light separation protocol, such as one or more dichroic mirrors, bandpass filters, diffraction gratings, beam splitters or prisms. In some embodiments, the wavelength separator is a prism. In other embodiments, the wavelength separator is a diffraction grating. In certain embodiments, wavelength separators in the subject light detection systems are dichroic mirrors.

In certain cases, one or more detectors in the system may be considered a trigger sensor (i.e., a sensor that observes the presence of the particle and produces a trigger signal). In some embodiments, the trigger sensor is a forward-scattered light detector (e.g., such as those described above). In other cases, the trigger sensor is an axial light loss (ALL) channel sensor. In such cases, the processor may be configured to calculate a trigger window based on the trigger signal, wherein the trigger window provides a time period during which the particle is expected to pass through a detection zone of the detector, and obtain the baseline noise level at time periods that are outside of the trigger window.

Suitable flow cytometry systems may include, but are not limited to those described in Ormerod (ed.), Flow Cytometry: A Practical Approach, Oxford Univ. Press (1997); Jaroszeski et al. (eds.), Flow Cytometry Protocols, Methods in Molecular Biology No. 91, Humana Press (1997); Practical Flow Cytometry, 3rd ed., Wiley-Liss (1995); Virgo, et al. (2012) Ann Clin Biochem. Jan;49(pt 1):17-28; Linden, et. al., Semin Throm Hemost. 2004 Oct;30(5):502-11; Alison, et al. J Pathol, 2010 Dec; 222(4):335-344; and Herbig, et al. (2007) Crit Rev Ther Drug Carrier Syst. 24(3):203-255; the disclosures of which are incorporated herein by reference. In certain instances, flow cytometry systems of interest include BD Biosciences FACSCanto^{™} flow cytometer, BD Biosciences FACSCanto^{™} II flow cytometer, BD Accuri^{™} flow cytometer, BD Accuri^{™} C6 Plus flow cytometer, BD Biosciences FACSCelesta^{™} flow cytometer, BD Biosciences FACSLyric^{™} flow cytometer, BD Biosciences FACSVerse^{™} flow cytometer, BD Biosciences FACSymphony^{™} flow cytometer, BD Biosciences LSRFortessa^{™} flow cytometer, BD Biosciences LSRFortessa^{™} X-20 flow cytometer, BD Biosciences FACSPresto^{™} flow cytometer, BD Biosciences FACSVia^{™} flow cytometer and BD Biosciences FACSCalibur^{™} cell sorter, a BD Biosciences FACSCount^{™} cell sorter, BD Biosciences FACSLyric^{™} cell sorter, BD Biosciences Via^{™} cell sorter, BD Biosciences Influx^{™} cell sorter, BD Biosciences Jazz^{™} cell sorter, BD Biosciences AriaTM cell sorter, BD Biosciences FACSAria^{™} II cell sorter, BD Biosciences FACSAria^{™} III cell sorter, BD Biosciences FACSAria^{™} Fusion cell sorter and BD Biosciences FACSMelody^{™} cell sorter, BD Biosciences FACSymphony^{™} S6 cell sorter or the like.

In some embodiments, the subject systems are flow cytometric systems, such those described in U.S. Patent Nos. 10,663,476; 10,620,111; 10,613,017; 10,605,713; 10,585,031; 10,578,542; 10,578,469; 10,481,074; 10,302,545; 10,145,793; 10,113,967; 10,006,852; 9,952,076; 9,933,341; 9,726,527; 9,453,789; 9,200,334; 9,097,640; 9,095,494; 9,092,034; 8,975,595; 8,753,573; 8,233,146; 8,140,300; 7,544,326; 7,201,875; 7,129,505; 6,821,740; 6,813,017; 6,809,804; 6,372,506; 5,700,692; 5,643,796; 5,627,040; 5,620,842; 5,602,039; 4,987,086; 4,498,766; the disclosures of which are herein incorporated by reference in their entirety. In some cases, flow cytometry systems of the disclosure are clustered wavelength division (CWD) systems. CWD systems are described in, for example, U.S. Patent Application Publication No. 2021/0247293; the disclosure of which is herein incorporated by reference in its entirety.

In certain instances, flow cytometry systems of the disclosure are configured for imaging particles in a flow stream by fluorescence imaging using radiofrequency tagged emission (FIRE), such as those described in Diebold, et al. Nature Photonics Vol. 7(10); 806-810 (2013) as well as described in U.S. Patent Nos. 9,423,353; 9,784,661; 9,983,132; 10,006,852; 10,078,045; 10,036,699; 10,222,316; 10,288,546; 10,324,019; 10,408,758; 10,451,538; 10,620,111; and U.S. Patent Publication Nos. 2017/0133857; 2017/0328826; 2017/0350803; 2018/0275042; 2019/0376895 and 2019/0376894 the disclosures of which are herein incorporated by reference.

### NON-TRANSITORY COMPUTER-READABLE STORAGE MEDIA

Aspects of the present disclosure further include non-transitory computer readable storage mediums having instructions for practicing the subject methods. Computer readable storage mediums may be employed on one or more computers for complete automation or partial automation of a system for practicing methods described herein. In certain embodiments, instructions in accordance with the method described herein can be coded onto a computer-readable medium in the form of "programming", where the term "computer readable medium" as used herein refers to any non-transitory storage medium that participates in providing instructions and data to a computer for execution and processing. Examples of suitable non-transitory storage media include a floppy disk, hard disk, optical disk, magneto-optical disk, CD-ROM, CD-R, magnetic tape, non-volatile memory card, ROM, DVD-ROM, Blue-ray disk, solid state disk, flash drive, and network attached storage (NAS), whether or not such devices are internal or external to the computer. A file containing information can be "stored" on computer readable medium, where "storing" means recording information such that it is accessible and retrievable at a later date by a computer. The computer-implemented method described herein can be executed using programming that can be written in one or more of any number of computer programming languages. Such languages include, for example, Java, Python, Visual Basic, and C++, as well as many others.

In some embodiments, computer readable storage media of interest include a computer program stored thereon, where the computer program when loaded on the computer includes instructions having an algorithm for categorizing the analyte data based on analyte features associated therewith by generating a predicted class for the analyte data using a decision tree ensemble; and refining the categorized analyte data based on the analyte features and the predicted class using a distance-based classification model to classify the analyte data.

### UTILITY

The subject particle analyzers, methods and computer systems find use in a variety of applications where it is desirable to analyze and, optionally, sort particle components in a sample in a fluid medium, such as a biological sample, and then store sorted products, e.g., for later use, such as therapeutic use. The present disclosure particularly finds use where it is desirable to classify (e.g., phenotype) flow cytometer data in a certain population of flow cytometer data. For example, the subject particle analyzers, methods and computer systems may be employed to facilitate the determination of a suitable gate for a particular population or subpopulation of flow cytometer data, especially in data sets where such suitable gates are not readily apparent. Embodiments of the disclosure also find use where it is desirable to provide a flow cytometer with improved cell sorting accuracy, enhanced particle collection, particle charging efficiency, more accurate particle charging and enhanced particle deflection during cell sorting.

Embodiments of the disclosure find use in applications where cells prepared from a biological sample may be desired for research, laboratory testing or for use in therapy. In some embodiments, the subject methods and devices may facilitate obtaining individual cells prepared from a target fluidic or tissue biological sample. For example, the subject methods and systems facilitate obtaining cells from fluidic or tissue samples to be used as a research or diagnostic specimen for diseases such as cancer. Likewise, the subject methods and systems may facilitate obtaining cells from fluidic or tissue samples to be used in therapy. Methods and devices of the present disclosure allow for separating and collecting cells from a biological sample (e.g., organ, tissue, tissue fragment, fluid) with enhanced efficiency and low cost as compared to traditional flow cytometry systems.

### KiTs

Aspects of the present disclosure further include kits, where kits include storage media such as a magneto-optical disk, CD-ROM, CD-R, magnetic tape, non-volatile memory card, ROM, DVD-ROM, Blue-ray disk, solid state disk, and network attached storage (NAS). Any of these program storage media, or others now in use or that may later be developed, may be included in the subject kits. In embodiments, the program storage media include instructions for classifying flow cytometer data via first and second gates. In embodiments, the instructions contained on computer readable media provided in the subject kits, or a portion thereof, can be implemented as software components of a software for analyzing data. In these embodiments, computer-controlled systems according to the instant disclosure may function as a software "plugin" for an existing software package (e.g., FlowJo^{®}).

In addition to the above components, the subject kits may further include (in some embodiments) instructions, e.g., for installing the plugin to the existing software package. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, and the like. Yet another form of these instructions is a computer readable medium, e.g., diskette, compact disk (CD), portable flash drive, and the like, on which the information has been recorded. Yet another form of these instructions that may be present is a website address which may be used via the internet to access the information at a removed site.

The following is presented by way of explanation and not by way of limitation.

### EXPERIMENTAL

### Example 1:

Data from an imaging flow cytometer was explored in an attempt to identify the best parameters to discriminate debris/small particles from single cells and multiplets. This algorithm aims to simplify the process and make it an automated step for the analyst allowing for the cleanup of their data without removing unnecessary events which can happen by manually drawing gates in analysis software.

### Import and Split the Data

The following steps were performed:
1. Begin by importing the data exported from FlowJo^{®} with cells labeled in the SpecimenlD column. Events labeled as 0, 1, 2 were considered as debris , singlets, and multiplets respectively.
2. Call train_test_split() with a test_size of 20%. Save the output into X_train , X_test , y_train , and y_test , respectively. Specify the random_state parameter.
3. Reshape the data.

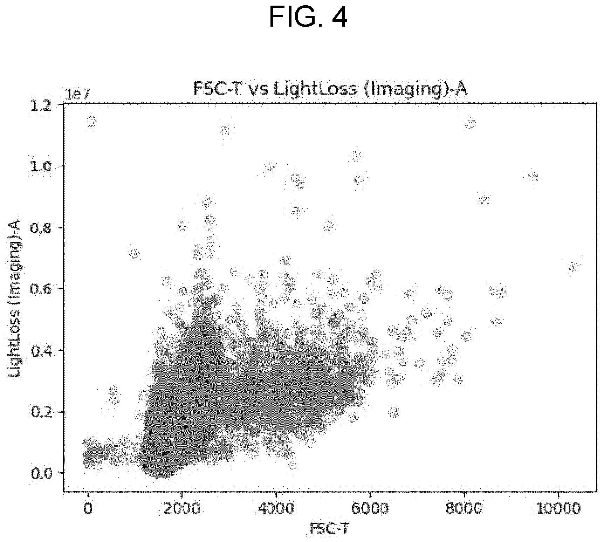

A resulting plot of the FSC-T vs LightLoss (Imaging)-A features outputted by the above-described process is depicted in **FIG. 4****.**

### Training and Test Sets

The train_test_split() function from scikit-learn was used to split a dataset into training and test sets. When one specifies an 80%-20% split, it means that 80% of the data will be used for training the model, and the remaining 20% will be used for testing it.

### Scaling the Data

StandardScalar is a preprocessing utility in the sklearn.preprocessing package. It standardizes features by subtracting the mean and scaling to unit variance. This is also known as z-score normalization or standardization. The following steps were performed:
1. Compute the mean and standard deviation For each feature column in the training dataset, the mean (average) and standard deviation are calculated.
2. Subtract the mean For each feature column in the training set, the mean is subtracted from the feature. This centers the feature around zero.
3. Divide by the standard deviation. For each feature in the training set, the result from step 2 is divided by the standard deviation. This scales the feature to have a standard deviation of 1.

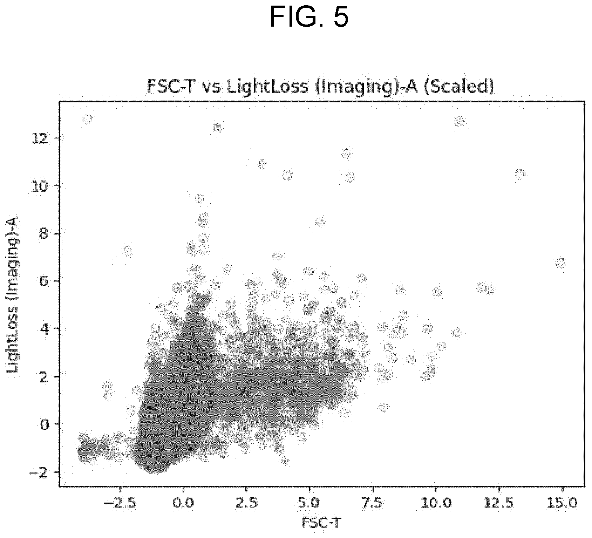

A resulting scaled plot of the FSC-T vs LightLoss (Imaging)-A features outputted by the above-described process is depicted in **FIG. 5****.**

After applying StandardScaler from scikit-learn, the features in the dataset were standardized, which means they were centered around the mean and scaled to unit variance. However, standardization does not guarantee that the features will have the same range or limits. The differences observed in the ranges can be due to several factors:
1. Original Distribution of the Data: If the original features have different distributions (e.g., one is normally distributed, and another is skewed), standardization will preserve these differences in distribution. Even though the features are scaled to have unit variance, their ranges can still be quite different if the original data was spread differently.
2. Outliers: Features with outliers might end up with a broader range after standardization. Since StandardScaler only scales the data, it doesn't handle outliers. Therefore, features with significant outliers will likely have a wider spread of standardized values.
3. Differences in Variance: Standardization scales the features to have unit variance, but if the original features have different variances, this can lead to different ranges in the scaled data. For instance, a feature with a larger variance in the original dataset might have a broader range after standardization.
4. Mean and Standard Deviation: StandardScaler uses the mean and standard deviation of each feature for scaling. If the original means and standard deviations are quite different across features, the scaled ranges can also differ.

### Visualization Aspect

When these standardized features are plotted, the scatter plot visualizes the data in terms of these new scales. The axes of the plot reflect the ranges of the standardized values, which, as mentioned, can differ across features. This is normal and expected when standardizing data that has varying distributions, variances, and outlier characteristics. The key point of StandardScaler was not to bring all features to the same range but to transform them in a way that their distribution is centered around zero and has a unit variance, making them more suitable for many machine learning algorithms.

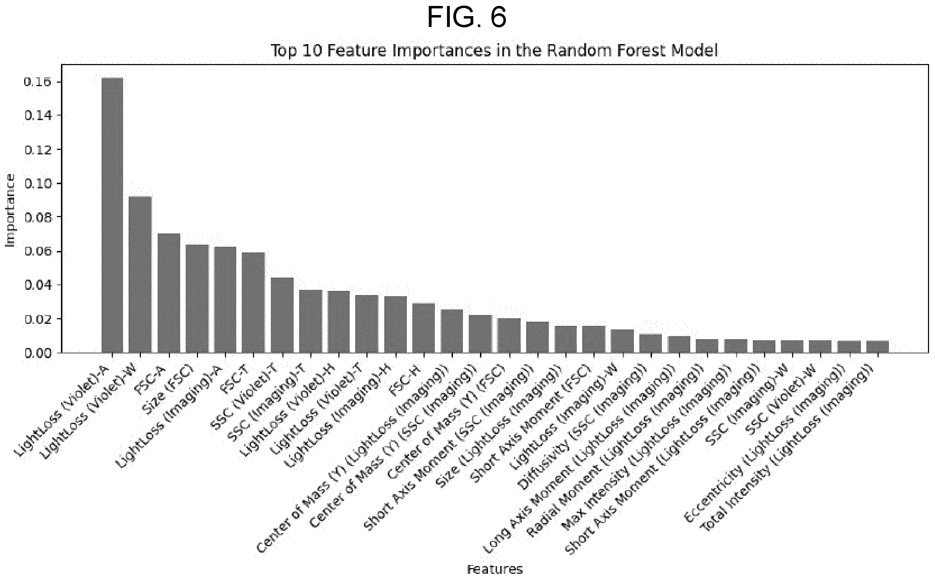

The .feature_importances_ attribute of a Random Forest model in scikit-learn provides a measure of the importance of each feature in the prediction. These importance values are a way to understand which features contribute most significantly to the model's decision-making process. A resulting plot of the top 10 feature importances in the random forest model is provided in **FIG. 6****.** The following results were obtained:
Total importance of the top 4 features: 0.388
Total importance of the top 5 features: 0.451
Total importance of the top 10 features: 0.661
Total importance of the top 15 features: 0.791
Total importance of the top 20 features: 0.866
Total importance of the top 25 features: 0.906
Total importance of the top 30 features: 0.939

### Example 2:

A random forest classification model was constructed by selecting the top 4 features, setting up a parameter grid for hyperparameter tuning, creating a grid search with random forest, fitting grid search to the reduced data, and finding the best parameters via the grid search. The following results were produced:
Best parameters found by Grid Search ROUND 1
Fitting 3 folds for each of 108 candidates, totaling 324 fits
{'max_depth': None,
'min_samples_leaf": 1,
'min_samples_split': 5,
'n_estimators': 200}
Best with top 25 features, 6min training time
{'max_depth': 30,
'min_samples_leaf": 1,
'min_samples_split': 2,
'n_estimators': 100}
Time to train ~2min20sec

The above was repeated for Round 2, with the following results:
Best parameters found by Grid Search ROUND 2
Fitting 3 folds for each of 108 candidates, totaling 324 fits
{'max_depth': None,
'min samples leaf': 1,
'min_samples_split': 6,
'n_estimators': 50}
Time to train ~3min22sec
Best with top 25 features, 3.5min training time
{'max_depth': 30,
# 'min_samples_leaf': 1,
# 'min_samples_split': 2,
# 'n_estimators': 100}

The above was repeated for Round 3, with the following results:
Best parameters found by Grid Search ROUND 3
Fitting 3 folds for each of 18 candidates, totaling 54 fits
{'max_depth': None,
'min_samples_leaf': 1,
'min_samples_split': 5,
'n_estimators': 250}
Time to train ~1min
Best with top 25 features, 3.5min training time
{'max_depth': 28,
'min_samples_leaf': 1,
'min_samples_split': 2,
'n_estimators': 85}

The above was repeated for Round 4, with similar results.

Below are the best parameters found after four rounds of hyperparameter tuning using the top four features.
{'max_depth': None, 'min_samples_leaf': 1, 'min_samples_split': 6, 'n_estimators': 48}
RandomForest training time: 10.414 seconds
Accuracy: 0.983
Classification Report:

| class | precision | recall | f1-score | support |
|---|---|---|---|---|
| Debris | 0.99 | 0.97 | 0.98 | 1089 |
| Single Cells | 0.99 | 0.99 | 0.99 | 5271 |
| Multiplets | 0.92 | 0.91 | 0.92 | 407 |
| accuracy | | | 0.98 | 6767 |
| macro avg | 0.96 | 0.96 | 0.96 | 6767 |
| weighted avg | 0.98 | 0.98 | 0.98 | 6767 |

### Using Top 4 Features

A model was built with the following values: best_params = {'max_depth': 20, 'min_samples_leaf': 2, 'min_samples_split': 2, 'n_estimators': 200}.

The following was observed:
Accuracy: 0.978276932170829
Classification report:

| class | precision | recall | f1-score | support |
|---|---|---|---|---|
| Debris | 0.98 | 0.97 | 0.98 | 1089 |
| Single Cells | 0.99 | 0.99 | 0.99 | 5271 |
| Multiplets | 0.87 | 0.89 | 0.88 | 407 |
| accuracy | | | 0.98 | 6767 |
| macro avg | 0.95 | 0.95 | 0.95 | 6767 |
| weighted avg | 0.98 | 0.98 | 0.98 | 6767 |

### Using Top 10 Features

Accuracy: 0.979
Classification report:

| class | precision | recall | f1-score | support |
|---|---|---|---|---|
| Debris | 0.98 | 0.97 | 0.98 | 1089 |
| Single Cells | 0.99 | 0.99 | 0.99 | 5271 |
| Multiplets | 0.88 | 0.89 | 0.89 | 407 |
| accuracy | | | 0.98 | 6767 |
| macro avg | 0.95 | 0.95 | 0.95 | 6767 |
| weighted avg | 0.98 | 0.98 | 0.98 | 6767 |

### Using Top 15 Features

Random Forest training time: 10.4 seconds
Accuracy: 0.983
Classification report:

| class | precision | recall | f1-score | support |
|---|---|---|---|---|
| Debris | 0.99 | 0.97 | 0.98 | 1089 |
| Single Cells | 0.99 | 0.99 | 0.99 | 5271 |
| Multiplets | 0.92 | 0.91 | 0.92 | 407 |
| accuracy | | | 0.98 | 6767 |
| macro avg | 0.96 | 0.96 | 0.96 | 6767 |
| weighted avg | 0.98 | 0.98 | 0.98 | 6767 |

### Using Top 20 Features

Accuracy: 0.982

| class | precision | recall | f1-score | support |
|---|---|---|---|---|
| Debris | 0.98 | 0.97 | 0.98 | 1089 |
| Single Cells | 0.99 | 0.99 | 0.99 | 5271 |
| Multiplets | 0.91 | 0.91 | 0.91 | 407 |
| accuracy | | | 0.98 | 6767 |
| macro avg | 0.96 | 0.96 | 0.96 | 6767 |
| weighted avg | 0.98 | 0.98 | 0.98 | 6767 |

### Using Top 25 Features

RandomForest training time: 10.598 seconds
Accuracy: 0.983

| class | precision | recall | f1-score | support |
|---|---|---|---|---|
| Debris | 0.99 | 0.97 | 0.98 | 1089 |
| Single Cells | 0.99 | 0.99 | 0.99 | 5271 |
| Multiplets | 0.92 | 0.91 | 0.92 | 407 |
| accuracy | | | 0.98 | 6767 |
| macro avg | 0.96 | 0.96 | 0.96 | 6767 |
| weighted avg | 0.98 | 0.98 | 0.98 | 6767 |

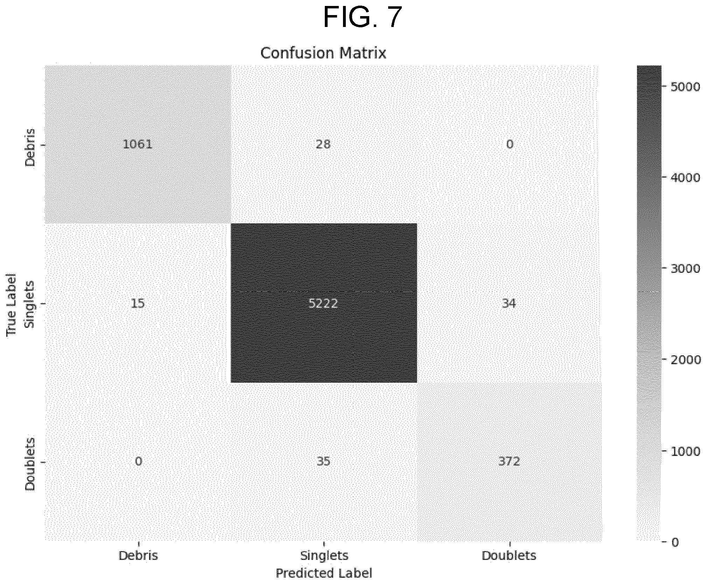

A confusion matrix of results was subsequently generated and is presented in **FIG. 7****.**

### Example 3:

Code was configured to select the top 10 features based on the ANOVA F-value, which measures the difference in means between groups relative to the variation within the groups. This is suitable for both positive and negative values. The following was produced:

```
 Selected features: Index(['FSC-A', 'SSC (Violet)-W', 'Center of Mass (Y) (LightLoss
      (Imaging))',
      'Center of Mass (Y) (SSC (Imaging))', 'LightLoss (Imaging)-A',
      'LightLoss (Imaging)-W', 'LightLoss (Violet)-A', 'LightLoss (Violet)-W',
      'Size (FSC)', 'Size (LightLoss (Imaging))'],
      dtype='object')
```

### Example 4:

There was an improvement above after using KNN but no hyperparameter tuning was performed. Parameters were tuned to squeeze more performance out of the models. This involved defining a parameter grid, initializing a grid search, fitting the grid search to the training data, printing the best parameters, and evaluating the best KNN model on the test data. Results for Round 1 are shown below:
Best parameters: {'knn_algorithm': 'ball_tree', 'knn_leaf_size': 15, 'knn_metric': 'manhattan', 'knn_n_neighbors': 3, 'knn_weights': 'distance'}
Best KNN Accuracy: 0.985

| class | precision | recall | f1-score | support |
|---|---|---|---|---|
| Debris | 0.99 | 0.98 | 0.98 | 1089 |
| Single Cells | 0.99 | 0.99 | 0.99 | 5271 |
| Multiplets | 0.93 | 0.90 | 0.91 | 407 |
| accuracy | | | 0.98 | 6767 |
| macro avg | 0.97 | 0.96 | 0.96 | 6767 |
| weighted avg | 0.98 | 0.98 | 0.98 | 6767 |

The above was repeated for Round 2, with the following results:
Best parameters: {'knn_algorithm': 'ball_tree', 'knn_leaf_size': 5, 'knn_metric': 'manhattan', 'knn_n_neighbors': 3,
'knn_weights': 'distance'}
Best KNN Accuracy: 0.985
Best KNN Classification Report:

| class | precision | recall | f1-score | support |
|---|---|---|---|---|
| Debris | 0.99 | 0.98 | 0.98 | 1089 |
| Single Cells | 0.99 | 0.99 | 0.99 | 5271 |
| Multiplets | 0.93 | 0.90 | 0.91 | 407 |
| accuracy | | | 0.98 | 6767 |
| macro avg | 0.97 | 0.96 | 0.96 | 6767 |
| weighted avg | 0.98 | 0.98 | 0.98 | 6767 |

The above was repeated for Round 3, with the following results:
Best KNN Accuracy: 0.985
# Best KNN Classification Report:

| class | precision | recall | f1-score | support |
|---|---|---|---|---|
| Debris | 0.99 | 0.98 | 0.98 | 1089 |
| Single Cells | 0.99 | 0.99 | 0.99 | 5271 |
| Multiplets | 0.93 | 0.90 | 0.91 | 407 |
| accuracy | | | 0.98 | 6767 |
| macro avg | 0.97 | 0.96 | 0.96 | 6767 |
| weighted avg | 0.98 | 0.98 | 0.98 | 6767 |

### Using Top 10 Features

When Testing KNN with the top ten features used to train the RF model above, the 'multiplets' class does not perform as well when all features are used to train and test KNN.
Best parameters: {'knn_metric': 'manhattan', 'knn_n_neighbors': 5, 'knn_weights': 'uniform'}
Best KNN Accuracy: 0.978
Classification Report:

| class | precision | recall | f1-score | support |
|---|---|---|---|---|
| Debris | 0.98 | 0.97 | 0.98 | 1089 |
| Single Cells | 0.99 | 0.99 | 0.99 | 5271 |
| Multiplets | 0.87 | 0.89 | 0.88 | 407 |
| accuracy | | | 0.98 | 6767 |
| macro avg | 0.95 | 0.95 | 0.95 | 6767 |
| weighted avg | 0.98 | 0.98 | 0.98 | 6767 |

### Using All Features

Best parameters: {'knn_metric': 'manhattan', 'knn_n_neighbors': 3, 'knn_weights': 'distance'}
Best KNN Accuracy: 0.985

| class | precision | recall | f1-score | support |
|---|---|---|---|---|
| Debris | 0.99 | 0.98 | 0.98 | 1089 |
| Single Cells | 0.99 | 0.99 | 0.99 | 5271 |
| Multiplets | 0.93 | 0.90 | 0.91 | 407 |
| accuracy | | | 0.98 | 6767 |
| macro avg | 0.97 | 0.96 | 0.96 | 6767 |
| weighted avg | 0.98 | 0.98 | 0.98 | 6767 |

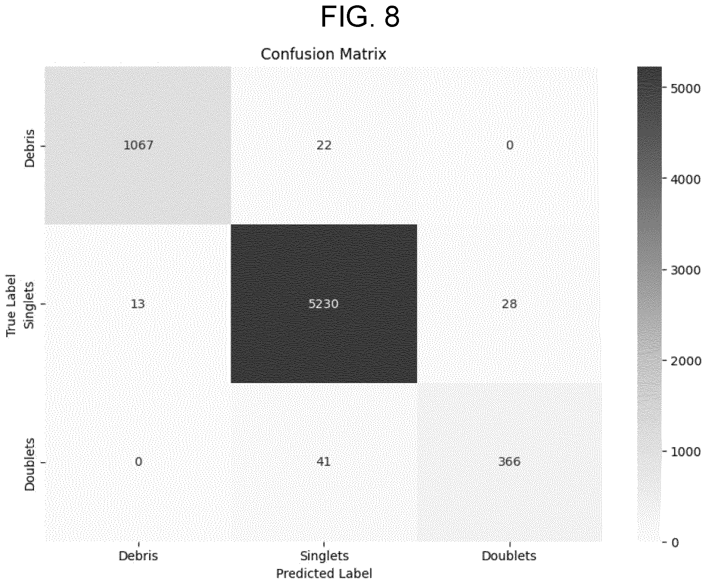

A confusion matrix of the best results from KNN gridsearch was generated, and the results are shown in **FIG. 8****.**

### Example 5:

The hyperparameter values found above to train the KNN model were used to predict the three classes (i.e., singlets, doublets, and debris). The results were compared to the random forest model from above:
KNN training time: 0.0483 seconds
KNN Accuracy: 0.983
KNN Classification Report:

| class | precision | recall | f1-score | support |
|---|---|---|---|---|
| Debris | 0.99 | 0.97 | 0.98 | 1089 |
| Single Cells | 0.99 | 0.99 | 0.99 | 5271 |
| Multiplets | 0.92 | 0.91 | 0.92 | 407 |
| accuracy | | | 0.98 | 6767 |
| macro avg | 0.96 | 0.96 | 0.96 | 6767 |
| weighted avg | 0.98 | 0.98 | 0.98 | 6767 |

### Example 6:

A custom classification model combining a random forest classification with KNN classification was built to gain benefits from both algorithms. This involved creating a "fit" definition for training the model. In this definition, top features were extracted for the random forest, which was then trained. Random forest predictions were obtained by making a copy of the new data frame, and saving predictions to a new column called "pureGate". The KNN was then trained on the features combined with the predictions.

The model also has a "predict" definition for classifying new data. In this definition, top features were extracted for the random forest. Then, the random forest was used to predict classifications of the data, which involved making a copy of the new data frame, saving predictions to a new column called "pureGate", and ensuring string column names. The features combined with the predictions were provided to the KNN, which then provided final predictions. The results were stored in a data frame with "pureGate" column name.

### Joint Tuning of Ensemble with Parallel Threads

Code was used to perform joint tuning with parallel threads for quick tuning times. This involved using a ThreadPoolExecutor to parallelize the evaluation of each combination of parameters in the grid search process. The best results were subsequently outputted:
Best Combination: (None, 4, 4, 150, 3, 'distance', 'manhattan')
Class-wise Precision: [0.98795181 0.98884688 0.9321608]
Class-wise Recall: [0.97887971 0.99241131 0.91154791]
Class-wise F1-Score: [0.98339483 0.99062589 0.92173913]

Ensemble model scores were then obtained after joint tuning using different numbers of features:

### All Features

Best Combination: (None, 1, 3, 80, 3, 'distance', 'manhattan')
Class-wise Precision: [0.98799631 0.9905321 0.93300248]
Class-wise Recall: [0.9825528 0.99241131 0.92383292]
Class-wise F1-Score: [0.98526703 0.99147081 0.92839506]

### Top 25 Features

Best Combination: (None, 1, 5, 80, 3, 'distance', 'manhattan')
Class-wise Precision: [0.98799631 0.99034274 0.93052109]
Class-wise Recall: [0.9825528 0.99222159 0.92137592]
Class-wise F1-Score: [0.98526703 0.99128127 0.92592593]

### Top 10 Features

Best Combination: (None, 1, 3, 80, 3, 'distance', 'manhattan')
Class-wise Precision: [0.98526703 0.98996212 0.93017456]
Class-wise Recall: [0.9825528 0.99165244 0.91646192]
Class-wise F1-Score: [0.98390805 0.99080656 0.92326733]

### Top 4 Features

Best Combination: (None, 2, 3, 100, 3, 'distance', 'manhattan')
Class-wise Precision: [0.98795181 0.98884055 0.92518703]
Class-wise Recall: [0.97887971 0.99184216 0.91154791]
Class-wise F1-Score: [0.98339483 0.99033908 0.91831683]

After joint tuning, the Best Score was 98.67% with the following hyperparameter settings:
Best Score: 0.987
Best Parameters: (Max Depth = None, Min Sam Leaf = 1, Min Samp Split = 6, N Estimators = 250, N_neighbors = 3, weights = 'distance', metric = 'manhattan')

The ensemble accuracy and classification report was generated and is shown below:
Ensemble Accuracy: 0.986

| class | precision | recall | f1-score | support |
|---|---|---|---|---|
| Debris | 0.99 | 0.98 | 0.98 | 1089 |
| Single Cells | 0.99 | 0.99 | 0.99 | 5271 |
| Multiplets | 0.93 | 0.92 | 0.92 | 407 |
| accuracy | | | 0.99 | 6767 |
| macro avg | 0.97 | 0.96 | 0.97 | 6767 |
| weighted avg | 0.99 | 0.99 | 0.99 | 6767 |

Using the Top Ten Features for RF model and all 50 (+1 additional classification result from RF predictions) for KNN, the results were improved. The KNN model results are shown below for comparison because they had the best precision, recall, and f1-score for the individually trained models:
Accuracy: 0.985
Classification Report:

| class | precision | recall | f1 -score | support |
|---|---|---|---|---|
| Debris | 0.99 | 0.98 | 0.98 | 1089 |
| Single Cells | 0.99 | 0.99 | 0.99 | 5271 |
| Multiplets | 0.93 | 0.91 | 0.92 | 407 |
| accuracy | | | 0.99 | 6767 |
| macro avg | 0.97 | 0.96 | 0.97 | 6767 |
| weighted avg | 0.99 | 0.99 | 0.99 | 6767 |

### Top 25 Parameters

Accuracy: 0.986
Classification Report:

| class | precision | recall | f1-score | support |
|---|---|---|---|---|
| Debris | 0.99 | 0.98 | 0.98 | 1089 |
| Single Cells | 0.99 | 0.99 | 0.99 | 5271 |
| Multiplets | 0.93 | 0.92 | 0.92 | 407 |
| accuracy | | | 0.99 | 6767 |
| macro avg | 0.97 | 0.96 | 0.97 | 6767 |
| weighted avg | 0.99 | 0.99 | 0.99 | 6767 |

### KNN

KNN Accuracy: 0.983
KNN Classification Report:

| class | precision | recall | f1-score | support |
|---|---|---|---|---|
| Debris | 0.99 | 0.97 | 0.98 | 1089 |
| Single Cells | 0.99 | 0.99 | 0.99 | 5271 |
| Multiplets | 0.92 | 0.92 | 0.92 | 407 |
| accuracy | | | 0.98 | 6767 |
| macro avg | 0.96 | 0.96 | 0.96 | 6767 |
| weighted avg | 0.98 | 0.98 | 0.98 | 6767 |

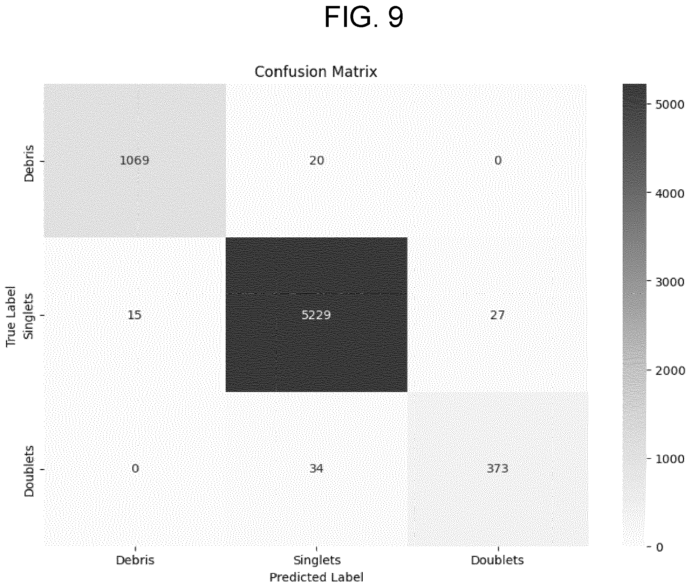

A confusion matrix generated using the ensemble method (i.e., combination of random forest and KNN) is shown in **FIG. 9****.** The results indicate performance improvements relative to random forest alone, or KNN alone, especially for the third class (i.e., row 2.0).

Notwithstanding the appended claims, the invention may also be defined by the following clauses:
1. A computer-implemented method of classifying analyte data, the method comprising, via a processor:
   categorizing the analyte data based on analyte features associated therewith by generating a predicted class for the analyte data using a decision tree ensemble; and
   refining the categorized analyte data based on the analyte features and the predicted class using a distance-based classification model to classify the analyte data.
2. The computer-implemented method according to Clause 1, wherein the decision tree ensemble is comprised of a random forest classification model.
3. The computer-implemented method according to Clause 1 or 2, wherein the distance-based classification model is a k-nearest neighbors classifier.
4. The computer-implemented method according to Clause 3, wherein *k* of the *k*-nearest neighbors classifier ranges from 2 to 4.
5. The computer-implemented method according to any one of the preceding clauses, wherein the distance of the distance-based classifier is selected from a Manhattan distance, a Euclidean distance, a Chebyshev distance and a cosine distance.
6. The computer-implemented method according to any one of Clauses 3 to 5, wherein the method comprises refining the predicted classes of the categorized analyte data using a vantage-point tree, a *k*-dimensional tree, ball tree, cover tree, locality-sensitive hashing, hierarchical navigable small world, approximate nearest neighbors with random projection trees, GPU-based KNN search, or a brute force KNN search.
7. The computer-implemented method according to any one of the preceding clauses, wherein the analyte data is flow cytometer data.
8. The computer-implemented method according to Clause 7, wherein the method comprises generating the flow cytometer data using a flow cytometer.
9. The computer-implemented method according to Clause 7 or 8, wherein the predicted class is selected from debris, single cells, and aggregates.
10. The computer implemented method according to any one of Clauses 7 to 9, wherein the analyte features are selected from size features, imaging features, and scatter features.
11. The computer-implemented method according to Clause 10, wherein the analyte features are scatter features selected from side-scatter (SSC) features and forward-scatter (FSC) features.
12. The computer implemented method according to any one of Clauses 7 to 11, wherein the analyte features comprise fluorescent features.
13. The computer implemented method according to Clause 12, further comprising classifying the analyte data into subgroups based on the fluorescent features.
14. The computer-implemented method according to any one of the preceding clauses, wherein the method comprises classifying the analyte data based on from 4 to 30 analyte features.
15. The computer-implemented method according to Clause 14, wherein the method comprises classifying the analyte data based on from 4 to 25 analyte features.
16. The computer-implemented method according to Clause 15, wherein the method comprises classifying the analyte data based on from 4 to 15 analyte features.
17. The computer-implemented method according to any one of the preceding clauses, further comprising ranking the analyte features by importance.
18. The computer-implemented method according to Clause 17, wherein ranking the analyte features by importance comprises calculating an ANOVA F-value.
19. The computer-implemented method according to any one of the preceding clauses, further comprising training the decision tree ensemble using analyte features from a training dataset.
20. The computer-implemented method according to Clause 19, further comprising training the distance-based classification model using the analyte features from the training dataset and the predicted class.
21. The computer-implemented method according to any one of the preceding clauses, further comprising producing an image of the classified analyte data.
22. The computer-implemented method according to Clause 21, wherein producing the image comprises rendering a gate around the classified analyte data.
23. The computer-implemented method according to any one of the preceding clauses, wherein the classified analyte data has an accuracy of greater than 0.95.
24. The computer-implemented method according to Clause 23, wherein the classified analyte data has an accuracy of greater than 0.97.
25. The computer-implemented method according to Clause 24, wherein the classified analyte data has an accuracy of greater than 0.98.
26. A system comprising memory operably coupled to a processor, wherein the memory comprises instructions stored thereon, which when executed by the processor, cause the processor to:
categorize analyte data based on analyte features associated therewith by generating a predicted class for the analyte data using a decision tree ensemble; and
refine the categorized analyte data based on the analyte features and the predicted class using a distance-based classification model to classify the analyte data.
27. The system according to Clause 26, wherein the decision tree ensemble is comprised of a random forest classification model.
28. The system according to Clause 26 or 27, wherein the distance-based classification model is a *k*-nearest neighbors classifier.
29. The system according to Clause 28, wherein *k* of the *k*-nearest neighbors classifier ranges from 2 to 4.
30. The system according to any one of Clauses 26 to 29, wherein the distance of the distance-based classifier is selected from a Manhattan distance, a Euclidean distance, a Chebyshev distance and a cosine distance.
31. The system according to any one of Clauses 28 to 30, wherein the processor is configured to refine the categorized analyte data using a vantage-point tree, a k-dimensional tree, ball tree, cover tree, locality-sensitive hashing, hierarchical navigable small world, approximate nearest neighbors with random projection trees, GPU-based KNN search, or a brute force KNN search.
32. The system according to any one of Clauses 26 to 31, wherein the analyte data is flow cytometer data.
33. The system according to Clause 32, further comprising a flow cytometer operably connected to the processor.
34. The system according to Clause 32 or 33, wherein the predicted class is selected from debris, single cells and aggregates.
35. The system according to any one of Clauses 32 to 34, wherein the analyte features are selected from size features, imaging features, and scatter features.
36. The system according to Clause 35, wherein the analyte features are scatter features selected from side-scatter (SSC) features and forward-scatter (FSC) features.
37. The system according to any one of Clauses 32 to 36, wherein the analyte features comprise fluorescent features.
38. The system according to Clause 37, wherein the processor is configured to classify the analyte data into subgroups based on the fluorescent features.
39. The system according to any one of Clauses 26 to 38, wherein processor is configured to classify the analyte data based on from 4 to 30 analyte features.
40. The system according to Clause 39, wherein the processor is configured to classify the analyte data based on from 4 to 25 analyte features.
41. The system according to Clause 40, wherein the processor is configured to classify the analyte data based on from 4 to 15 analyte features.
42. The system according to any one of Clauses 26 to 41, wherein the processor is configured to rank the analyte features by importance.
43. The system according to Clause 42, wherein ranking the analyte features by importance comprises calculating an ANOVA F-value.
44. The system according to any one of Clauses 26 to 43, wherein the processor is configured to train the decision tree ensemble using analyte features from a training dataset.
45. The system according to Clause 44, wherein the processor is configured to train the distance-based classification model using the analyte features from the training dataset and the predicted class.
46. The system according to any one of Clauses 26 to 45, wherein the processor is configured to produce an image of the classified analyte data.
47. The system according to Clause 46, wherein producing the image comprises rendering a gate around the classified analyte data.
48. The system according to any one of Clauses 26 to 47, wherein the classified analyte data has an accuracy of greater than 0.95.
49. The system according to Clause 48, wherein the classified analyte data has an accuracy of greater than 0.97.
50. The system according to Clause 49, wherein the classified analyte data has an accuracy of greater than 0.98.
51. A non-transitory computer-readable storage medium comprising instructions stored thereon for classifying analyte data by a method comprising:
categorizing the analyte data based on analyte features associated therewith by generating a predicted class for the analyte data using a decision tree ensemble; and
refining the categorized analyte data based on the analyte features and the predicted class using a distance-based classification model to classify the analyte data.
52. The non-transitory computer-readable storage medium according to Clause 51, wherein the decision tree ensemble is comprised of a random forest classification model.
53. The non-transitory computer-readable storage medium according to Clause 51 or 52, wherein the distance-based classification model is a k-nearest neighbors classifier.
54. The non-transitory computer-readable storage medium according to Clause 53, wherein *k* of the *k*-nearest neighbors classifier ranges from 2 to 4.
55. The non-transitory computer-readable storage medium according to any one of Clauses 51 to 54, wherein the distance of the distance-based classifier is selected from a Manhattan distance, a Euclidean distance, a Chebyshev distance and a cosine distance.
56. The non-transitory computer-readable storage medium according to any one of Clauses 53 to 55, wherein the method comprises refining the categorized analyte data using a vantage-point tree, a k-dimensional tree, ball tree, cover tree, locality-sensitive hashing, hierarchical navigable small world, approximate nearest neighbors with random projection trees, GPU-based KNN search, or a brute force KNN search.
57. The non-transitory computer-readable storage medium according to any one of Clauses 51 to 56, wherein the analyte data is flow cytometer data.
58. The non-transitory computer-readable storage medium according to Clause 57, wherein the method comprises generating the flow cytometer data using a flow cytometer.
59. The non-transitory computer-readable storage medium according to Clause 57 or 58, wherein the predicted class is selected from debris, single cells and aggregates.
60. The non-transitory computer-readable storage medium according to any one of Clauses 57 to 59, wherein the analyte features are selected from size features, imaging features, and scatter features.
61. The non-transitory computer-readable storage medium according to Clause 60, wherein the analyte features are scatter features selected from side-scatter (SSC) features and forward-scatter (FSC) features.
62. The non-transitory computer-readable storage medium according to any one of Clauses 57 to 61, wherein the analyte features comprise fluorescent features.
63. The non-transitory computer-readable storage medium according to Clause 62, wherein the method further comprises classifying the analyte data into subgroups based on the fluorescent features.
64. The non-transitory computer-readable storage medium according to any one of Clauses 51 to 63, wherein the method comprises classifying the analyte data based on from 4 to 30 analyte features.
65. The non-transitory computer-readable storage medium according to Clause 64, wherein the method comprises classifying the analyte data based on from 4 to 25 analyte features.
66. The non-transitory computer-readable storage medium according to Clause 65, wherein the method comprises classifying the analyte data based on from 4 to 15 analyte features.
67. The non-transitory computer-readable storage medium according to any one of Clauses 51 to 66, wherein the method further comprises ranking the analyte features by importance.
68. The non-transitory computer-readable storage medium according to Clause 67, wherein ranking the analyte features by importance comprises calculating an ANOVA F-value.
69. The non-transitory computer-readable storage medium according to any one of Clauses 51 to 68, wherein the method further comprises training the decision tree ensemble using analyte features from a training dataset.
70. The non-transitory computer-readable storage medium according to Clause 69, wherein the method further comprises training the distance-based classification model using the analyte features from the training dataset and the predicted class.
71. The non-transitory computer-readable storage medium according to any one of Clauses 51 to 70, wherein the method further comprises producing an image of the classified analyte data.
72. The non-transitory computer-readable storage medium according to Clause 71, wherein producing the image comprises rendering a gate around the classified analyte data.
73. The non-transitory computer-readable storage medium according to any one of Clauses 51 to 72, wherein the classified analyte data has an accuracy of greater than 0.95.
74. The non-transitory computer-readable storage medium according to Clause 73, wherein the classified analyte data has an accuracy of greater than 0.97.
75. The non-transitory computer-readable storage medium according to Clause 74, wherein the classified analyte data has an accuracy of greater than 0.98.
76. A computer-implemented method of classifying analyte data, the method comprising:
   (a) providing analyte data to a system comprising memory operably coupled to the processor, wherein the memory comprises instructions stored thereon, which when executed by the processor, cause the processor to:
      categorize the analyte data based on analyte features associated therewith by generating a predicted class for the analyte data using a decision tree ensemble; and
      refine the categorized analyte data based on the analyte features and the predicted class using a distance-based classification model to classify the analyte data; and
   (b) receiving the classified analyte data from the processor.
77. The computer-implemented method according to Clause 76, wherein the decision tree ensemble is comprised of a random forest classification model.
78. The computer-implemented method according to Clause 76 or 77, wherein the distance-based classification model is a *k*-nearest neighbors classifier.
79. The computer-implemented method according to Clause 78, wherein *k* of the *k*-nearest neighbors classifier ranges from 2 to 4.
80. The computer-implemented method according to any one of Clauses 76 to 79, wherein the distance of the distance-based classifier is selected from a Manhattan distance, a Euclidean distance, a Chebyshev distance and a cosine distance.
81. The computer-implemented method according to any one of Clauses 78 to 80, wherein the processor is configured to refine the categorized analyte data using a vantage-point tree, a *k*-dimensional tree, ball tree, cover tree, locality-sensitive hashing, hierarchical navigable small world, approximate nearest neighbors with random projection trees, GPU-based KNN search, or a brute force KNN search.
82. The computer-implemented method according to any one of Clauses 76 to 81, wherein the analyte data is flow cytometer data.
83. The computer-implemented method according to Clause 82, wherein the system further comprises a flow cytometer operably connected to the processor.
84. The computer-implemented method according to Clause 82 or 83, wherein the predicted class is selected from debris, single cells, and aggregates.
85. The computer-implemented method according to any one of Clauses 82 to 84, wherein the analyte features are selected from size features, imaging features, and scatter features.
86. The computer-implemented method according to Clause 85, wherein the analyte features are scatter features selected from side-scatter (SSC) features and forward-scatter (FSC) features.
87. The computer-implemented method according to any one of Clauses 82 to 86, wherein the analyte features comprise fluorescent features.
88. The computer-implemented method according to Clause 87, wherein the processor is configured to classify the analyte data into subgroups based on the fluorescent features.
89. The computer-implemented method according to any one of Clauses 76 to 88, wherein processor is configured to classify the analyte data based on from 4 to 30 analyte features.
90. The computer-implemented method according to Clause 89, wherein processor is configured to classify the analyte data based on from 4 to 25 analyte features.
91. The computer-implemented method according to Clause 90, wherein processor is configured to classify the analyte data based on from 4 to 15 analyte features.
92. The computer-implemented method according to any one of Clauses 76 to 91, wherein the processor is configured to rank the analyte features by importance.
93. The computer-implemented method according to Clause 92, wherein ranking the analyte features by importance comprises calculating an ANOVA F-value.
94. The computer-implemented method according to any one of Clauses 76 to 93, wherein the processor is configured to train the decision tree ensemble using analyte features from a training dataset.
95. The computer-implemented method according to Clause 94, wherein the processor is configured to train the distance-based classification model using the analyte features from the training dataset and the predicted class.
96. The computer-implemented method according to any one of Clauses 76 to 95, wherein the processor is configured to produce an image of the classified analyte data.
97. The computer-implemented method according to Clause 96, wherein producing the image comprises rendering a gate around the classified analyte data.
98. The computer-implemented method according to any one of Clauses 76 to 97, wherein the classified analyte data has an accuracy of greater than 0.95.
99. The computer-implemented method according to Clause 98, wherein the classified analyte data has an accuracy of greater than 0.97.
100. The computer-implemented method according to Clause 99, wherein the classified analyte data has an accuracy of greater than 0.98.

Although the foregoing disclosure has been described in some detail by way of illustration and example for purposes of clarity of understanding, it is readily apparent to those of ordinary skill in the art in light of the teachings of this disclosure that some changes and modifications may be made thereto without departing from the spirit or scope of the appended claims.

Accordingly, the preceding merely illustrates the principles of the disclosure. It will be appreciated that those skilled in the art will be able to devise various arrangements which, although not explicitly described or shown herein, embody the principles of the disclosure and are included within its spirit and scope. Furthermore, all examples and conditional language recited herein are principally intended to aid the reader in understanding the principles of the disclosure and the concepts contributed by the inventors to furthering the art, and are to be construed as being without limitation to such specifically recited examples and conditions. Moreover, all statements herein reciting principles, aspects, and embodiments of the disclosure as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.

The scope of the present disclosure, therefore, is not intended to be limited to the exemplary embodiments shown and described herein. Rather, the scope and spirit of present disclosure is embodied by the appended claims. In the claims, 35 U.S.C. §112(f) or 35 U.S.C. §112(6) is expressly defined as being invoked for a limitation in the claim only when the exact phrase "means for" or the exact phrase "step for" is recited at the beginning of such limitation in the claim; if such exact phrase is not used in a limitation in the claim, then 35 U.S.C. § 112 (f) or 35 U.S.C. §112(6) is not invoked.

## Claims

1. A computer-implemented method of classifying analyte data, the method comprising, via a processor:
categorizing the analyte data based on analyte features associated therewith by generating a predicted class for the analyte data using a decision tree ensemble; and
refining the categorized analyte data based on the analyte features and the predicted class using a distance-based classification model to classify the analyte data.

2. The computer-implemented method according to Claim 1, wherein the decision tree ensemble is comprised of a random forest classification model.

3. The computer-implemented method according to Claim 1 or 2, wherein the distance-based classification model is a k-nearest neighbors classifier.

4. The computer-implemented method according to any one of the preceding claims, wherein the distance of the distance-based classifier is selected from a Manhattan distance, a Euclidean distance, a Chebyshev distance and a cosine distance.

5. The computer-implemented method according to any one of the preceding claims, wherein the analyte data is flow cytometer data.

6. The computer implemented method according to Claim 5, wherein the analyte features comprise fluorescent features.

7. The computer implemented method according to Claim 6, further comprising classifying the analyte data into subgroups based on the fluorescent features.

8. The computer-implemented method according to any one of the preceding claims, wherein the method comprises classifying the analyte data based on from 4 to 30 analyte features.

9. The computer-implemented method according to any one of the preceding claims, further comprising ranking the analyte features by importance.

10. The computer-implemented method according to any one of the preceding claims, further comprising training the decision tree ensemble using analyte features from a training dataset.

11. The computer-implemented method according to any one of the preceding claims, further comprising producing an image of the classified analyte data.

12. The computer-implemented method according to any one of the preceding claims, wherein the classified analyte data has an accuracy of greater than 0.95.

13. A system comprising memory operably coupled to a processor, wherein the memory comprises instructions stored thereon, which when executed by the processor, cause the processor to:
categorize analyte data based on analyte features associated therewith by generating a predicted class for the analyte data using a decision tree ensemble; and
refine the categorized analyte data based on the analyte features and the predicted class using a distance-based classification model to classify the analyte data.

14. A non-transitory computer-readable storage medium comprising instructions stored thereon for classifying analyte data by a method comprising:
categorizing the analyte data based on analyte features associated therewith by generating a predicted class for the analyte data using a decision tree ensemble; and
refining the categorized analyte data based on the analyte features and the predicted class using a distance-based classification model to classify the analyte data.

15. A computer-implemented method of classifying analyte data, the method comprising:
(a) providing analyte data to a system comprising memory operably coupled to the processor, wherein the memory comprises instructions stored thereon, which when executed by the processor, cause the processor to:
categorize the analyte data based on analyte features associated therewith by generating a predicted class for the analyte data using a decision tree ensemble; and
refine the categorized analyte data based on the analyte features and the predicted class using a distance-based classification model to classify the analyte data; and
(b) receiving the classified analyte data from the processor.
